# EUROPEAN PATENT APPLICATION

(11) **EP 4 414 390 A1**
(43) Date of publication of application: **14.08.2024**
(21) Application number: 23156186.1
(22) Date of filing: 10.02.2023
(51) Int. Cl.: C08F 4/34, C08F 4/40, C08F 220/18, C08F 222/10

(54) **ACYLTHIOUREA OLIGOMERS SUITABLE AS REDOX INITIATOR COMPONENTS AND RADICALLY POLYMERIZABLE COMPOSITIONS COMPRISING THEM**

(71) Applicant: Ivoclar Vivadent AG, 9494 Schaan (LI)
(72) Inventor: Catel, Yohann, 9475 Sevelen (CH); Lamparth, Iris, 9472 Grabs (CH); Fässler, Pascal, 8887 Mels (CH); Schnur, Thomas, 9488 Schellenberg (DE)
(74) Representative: Uexküll & Stolberg

(57) **Abstract**

Acylthiourea oligomers according to the formula

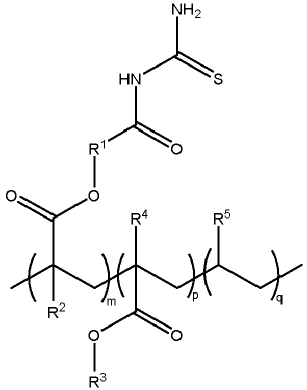

The acylthiourea oligomers, in combination with a hydroperoxide compound, are active as initiators for the radical polymerization. Radically polymerizable compositions comprising at least one acylthiourea, at least one hydroperoxide and at least one radically polymerizable monomer are particularly suitable as dental materials.

## Description

The present invention relates to acylthiourea oligomers which in combination with hydroperoxides are active as initiators for the free radical polymerization, and to radically polymerizable compositions comprising the same. The oligomers are particularly suitable for the production of dental materials, e.g. luting materials, filling composites and temporary filling materials.

In the dental field, composites are mainly used for the fabrication of direct restorations or for the cementation of indirect restorations. The polymerizable organic matrix of the composites usually consists of a mixture of monomers, initiator components, pigments and stabilizers, with mixtures of dimethacrylates often being used as monomers. Such materials can be cured by thermal, redox-initiated or light-induced radical polymerization. In addition to the organic matrix, composites contain one or more fillers, which are usually surface-modified with a polymerizable coupling agent, such as 3-methacryloyloxypropyltrimethoxysilane. Fillers improve the mechanical properties (strength, modulus of elasticity, abrasion resistance) and the processing properties (paste consistency, sculptability) of the materials and impart radiopacity.

Methacrylate-based dental materials are cured by radical polymerization using radical photoinitiators or redox initiator systems, depending on the field of application. Dual-curing systems contain a combination of photoinitiators and redox initiators.

Common photoinitiators are α-diketones such as e.g. camphorquinone (1,7,7-tri-methylbicyclo[2.2.1]heptane-2,3-dione; CQ) and 9,10-phenanthrenequinone which are often used in combination with a coinitiator.

Redox initiator systems are used in dentistry to enable the self-cure of dental materials such as luting materials, composites, and temporary materials. The initiators are key components of luting materials, which are required to mediate a bond between the tooth substance and indirect restorations, such as ceramic restorations. Luting composites and self-adhesive composite cements are among the most commonly used luting materials in dentistry. These materials comprise an organic matrix, usually a mixture of dimethacrylates, an initiator system, additives, and inorganic fillers such as silanized glass fillers, spherical mixed oxides, ytterbium fluoride, etc. They are mostly available as light-curing (LC) or dual-curing (DC) materials. Dual-curing is essential if not enough light can reach the cement through the restoration, e.g. due to its opacity, shade or thickness. The initiator system of DC luting materials contains both a photoinitiator and a redox initiator.

To ensure adequate storage stability of the redox initiators, redox initiator system-based materials are usually used as so-called 2-component (2C) systems, in which the components of the redox initiator, i.e. oxidizing agent, e.g. peroxide or hydroperoxide, and reducing agent, e.g. amines, sulfinic acids, barbiturates, thioureas, etc., are incorporated into separate components. These are mixed together just before use. For mixing, double-push syringes are increasingly being used, which have separate cylindrical chambers for holding the components. The components are pressed out of the chambers simultaneously with two connected pistons and mixed in a nozzle. To obtain mixtures that are as homogeneous as possible, it is advantageous to mix the components in approximately equal proportions by volume.

The first paste of two-component DC cements typically contains the oxidizing agent, while the second paste contains a reducing agent, and often also a metal catalyst. Upon mixing of the two pastes, a redox reaction takes place, generating radicals that initiate polymerization. The working time of the material is *inter alia* determined by the reactivity of the redox initiator system. The working time should not be too short so that the dentist can carry out the cementation before the material hardens. However, long working times are also not desirable in order to avoid unnecessary waiting time in the dental practice.

Redox-initiator systems are also used in temporary dental materials as well as in DC flowable composites for the direct filling therapy. The main advantage of DC composites is their unlimited depth of cure, as light is not mandatory for the curing of the material.

Redox initiator systems based on a mixture of dibenzoyl peroxide (DBPO) with tertiary aromatic amines, such as 4-(N,N-dimethylamino)benzoic acid ethyl ester (EDMAB), N,N-diethanol-p-toluidine (DEPT), N,N-dimethyl-sym.-xylidine (DMSX) or N,N-diethyl-3,5-di-tert-butylaniline (DABA) have been widely used to initiate radically polymerization of two-component dental materials. However, this initiator system has major drawbacks. Due to the thermal instability of DBPO, dental materials containing this peroxide must be stored in the refrigerator. In addition, the radical formation using DBPO/amine-based redox initiator systems is greatly impaired by strong acids and thus also by strongly acidic adhesive monomers which react with the amine.

Moreover, oxidation of the amine can lead to discolorations of the materials. Materials comprising amines are therefore unstable.

Consequently, the dental industry intensively searched for alternative redox initiator systems that would allow the formulation of materials that can be stored at room temperature. Nowadays, hydroperoxides, such as cumene hydroperoxide (CHP) or amyl hydroperoxide, are mostly used as oxidizing agents in DC dental materials. Such hydroperoxides exhibit significantly improved thermal stability and can be stored at room temperature. On the other hand, they do not react efficiently with tertiary aromatic amines, and therefore alternative reducing agents are needed.

During the past decades, reducing agents such as (thio)barbituric acid, sulfinic acid, and ascorbic acid derivatives have been evaluated in dental materials (EP 0 480 785 B1, US 6,953,535 B2, US 7,465,758 B2, EP 0 408 357 B1, US 10,932,994 B2). These reducing agents are, however, sensitive to oxygen and it is challenging to formulate storage stable materials based thereon.

For this reason, technologies based on the corresponding salts, such as sodium, calcium, potassium, ammonium salts, have been proposed (US 8,236,871 B2). These salts are dispersed in one component of the DC cement, and an acid is added to the second component. After mixing of both components, the effective reducing agent is generated via the protonation of the corresponding salt, leading to an initiation of the polymerization.

Another redox initiator system that has been developed and used in dental materials involves (acyl)thioureas (US 7,541,393 B2). Redox-initiator systems based on thiourea derivatives exhibit good acid-stability. In particular, combinations of a thiourea derivative, such as acetylthiourea, with a hydroperoxide, such as cumene hydroperoxide, are stable in the presence of strongly acidic adhesive monomers.

EP 1 693 046 A1 discloses a dental composition that is said to be compatible with acidic dental primers/adhesives. The composition contains a redox initiator system that results from the combination of a (2-pyridyl)-2-thiourea derivative with a hydroperoxide compound.

WO 2007/016508 A1 discloses dental compositions that contain a thiourea/hydroperoxide combination as an initiator system. Those compositions do not contain acid-functional compounds.

According to EP 1 754 465 A1, the reactivity of the thiourea/hydroperoxide system can be increased by adding a soluble copper compound.

EP 2 233 544 B1 describes two-component dental compositions that contain a redox initiator system based on the combination of a thiourea derivative, a hydroperoxide and a vanadium compound as a polymerization accelerator.

Although DC dental materials based on the thiourea and hydroperoxide system are storage stable, they still have drawbacks. In particular, thiourea derivatives have a bitter taste (D. Mela, Chem. Senses 14 (1989) 131-135; Lange et al., Chem. Amer. Chem. Soc. 51 (1929) 1911-1914; Qin et al., Talanta 199 (2019) 131-139) that can still be perceived after curing, which many patients find unpleasant.

EP 4 039 220 A1 discloses radically polymerizable dental materials wherein the redox system comprises a hydroperoxide and an acylthiourea derivative comprising a radically polymerizable group. The materials can additionally contain a transition metal compound. On curing, the acylthiourea derivatives are incorporated into the polymer network. If the acylthiourea derivatives do not polymerize completely, residual monomers can be leached out of the material and cause a bitter taste.

It is an object of the invention to provide radically polymerizable materials which do not have the disadvantages of the state of the art and which are particularly suitable as dental materials. The materials must not taste bitter and should have good biocompatibility. In addition, the materials should exhibit excellent mechanical properties (flexural strength and flexural modulus) as well as an appropriate working time.

These objects are achieved by radically polymerizable compositions comprising at least one acylthiourea oligomer according to Formula (I):

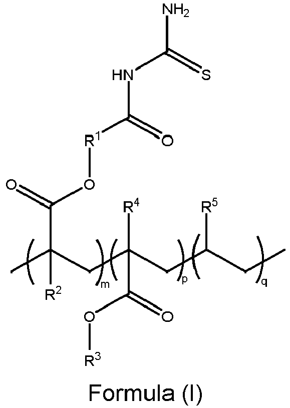

It was found that acylthiourea oligomers according to Formula (I) do not have a bitter taste and in combination with a hydroperoxide are active as initiators for free radical polymerization.

The variables of Formula (I) have the following meanings:
- R¹: is a branched or a linear C₁-C₁₆ alkanediyl group, preferably a C₁-C₁₂ alkanediyl group, and more preferably a C₁-C₈ alkanediyl group, that can be interrupted by one or more S atoms, O atoms, -C(O)O- groups and/or -C(O)NH- groups;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and can be interrupted by one or more S atoms, O atoms, -C(O)O- groups and/or -C(O)NH- groups;
   or
a combination of the branched or the linear C₁-C₁₆ alkanediyl group and the C₄-C₁₆ cycloaliphatic group;
   or
a C₇-C₂₀-alkylaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R⁸, where R⁸ is a branched or preferably linear C₁-C₂₀ alkyl group, and/or acyl groups -C(O)-R⁹, where R⁹ is a branched or preferably linear C₁-C₂₀ alkyl group;
- R², R⁴: are, independently of each other, hydrogen or a methyl group;
- R³: is a branched or preferably linear C₁-C₁₂ alkyl group, preferably a C₁-C₁₀ alkyl group, and more preferably a C₁-C₈ alkyl group, that can be interrupted by one or more S atoms, O atoms, -C(O)O- groups and/or -C(O)NH- groups and/or can be substituted by one or more functional groups, preferably OH, COOH and/or halogen;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, that can be interrupted by one or more S atoms, O atoms, -C(O)O- groups and/or C(O)NH- groups;
   or
a C₄-C₁₀ aryl or heteroaryl group which can be unsubstituted or substituted by one or more substituents which are selected from branched or preferably linear C₁-C₂₀ alkyl groups, -O-C₁-C₁₂ alkoxy groups, ester groups -C(O)O-R¹⁰, where R¹⁰ is a branched or preferably linear C₁-C₂₀ alkyl group, a C₆-C₁₀ aryl group or a C₇-C₂₀-alkylaryl group, and/or acyl groups -C(O)-R¹¹, where R¹¹ is a branched or preferably linear C₁-C₂₀ alkyl group, a C₆-C₁₀ aryl group or a C₇-C₂₀-alkylaryl group and/or can be interrupted by one or more S atoms or O atoms;
   or
a C₅-C₂₀-alkylaryl or C₅-C₂₀-alkylheteroaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, branched or preferably linear O-C₁-C₁₂ alkoxy groups, ester groups -C(O)O-R¹², where R¹² is a branched or preferably linear C₁-C₂₀ alkyl group, a C₆-C₁₀ aryl group or a C₇-C₂₀-alkylaryl group, and/or acyl groups -C(O)-R¹³, where R¹³ is a branched or preferably linear C₁-C₂₀ alkyl group, a C₆-C₁₀ aryl group or a C₇-C₂₀-alkylaryl group;
- R⁵: is a phenyl or a -O(C=O)-CH₃ group;
- m: is an integer within a range from 1 to 100, preferably 2 to 50, most preferably 2 to 30;
- p: is an integer within a range from 0 to 100, preferably 2 to 50, most preferably 5 to 50;
- q: is an integer within a range from 0 and 100, preferably 0 to 50, most preferably 0 to 30;
m + p + q ≥ 2 and m/(m + p + q) is a value within a range from 0.1 and 1.0, preferably 0.1 to 0.5, most preferably 0.2 to 0.5.

Acylthiourea oligomers according to Formula (I) wherein q = 0 are preferred according to the present invention. Such oligomers can be represented by Formula (II):

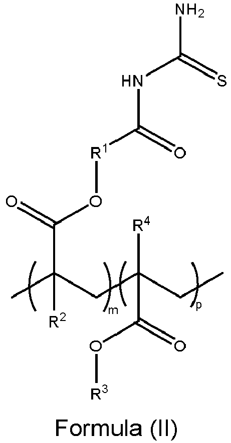

Particularly preferred are compounds according to Formula (II) wherein the variables have the following meanings:
- R¹: is a branched or a linear C₁-C₁₆ alkanediyl group, preferably a C₁-C₁₂ alkanediyl group, and more preferably a C₁-C₈ alkanediyl group, that can be interrupted by one or more S atoms, O atoms, -C(O)O- groups and/or -C(O)NH- groups;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and can be interrupted by one or more S atoms, O atoms, -C(O)O- groups and/or -C(O)NH- groups;
or a combination of branched or the linear C₁-C₁₆ alkanediyl group and the C₄-C₁₆ cycloaliphatic group;
   or
a C₇-C₂₀-alkylaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, branched or preferably linear O-C₁-C₁₂ alkoxy groups, ester groups -C(O)O-R⁸, where R⁸ is a branched or preferably linear C₁-C₂₀ alkyl group, and/or acyl groups -C(O)-R⁹, where R⁹ is a branched or preferably linear C₁-C₂₀ alkyl group;
- R²: is hydrogen atom or a methyl group;
- R³: is a branched or preferably linear C₁-C₁₂ alkyl group, preferably a C₁-C₁₀ alkyl group, and more preferably a C₁-C₈ alkyl group, that can be interrupted by one or O atoms, -C(O)O- groups and/or -C(O)NH- groups and/or that can be substituted by one or more functional groups, preferably OH, COOH and/or halogen;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and can be interrupted by one or more S atoms, O atoms, -C(O)O- groups and/or -C(O)NH- groups;
   or
a C₄-C₁₀ aryl or heteroaryl group which can be unsubstituted or substituted by one or more substituents selected from branched or preferably linear C₁-C₂₀ alkyl groups, -O-C₁-C₁₂ alkoxy groups, ester groups -C(O)O-R¹⁰, where R¹⁰ is either a branched or preferably linear C₁-C₂₀ alkyl group, and/or acyl groups -C(O)-R¹¹, where R¹¹ is a branched or preferably a linear C₁-C₂₀ alkyl group;
   or
a C₅-C₂₀-alkylaryl or alkylheteroaryl group that can be interrupted by one or more O atoms, -C(O)O- groups and/or -C(O)NH- groups, and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R¹², where R¹² is a branched or preferably linear C₁-C₂₀ alkyl group, and/or acyl groups -C(O)-R¹³, where R¹³ is a branched or preferably linear C₁-C₂₀ alkyl group;
- R⁴: is a hydrogen atom or a methyl group;
- m: is an integer within a range from 1 to 100, preferably 2 to 50, most preferably 2 to 30;
- p: is an integer within a range from 0 to 100, preferably 2 to 50, most preferably 5 and 50;

m + p ≥ 2 and
m/(m+p) is a value within the range from 0.1 to 1.0, preferably 0.1 to 0.5, most preferably 0.2 to 0.5.

More preferably the variables of Formula (II) have the following meanings:
- R¹: is a branched or a linear C₁-C₁₆ alkanediyl group, preferably a C₁-C₁₂ alkanediyl group, and more preferably a C₁-C₈ alkanediyl group, that can be interrupted by one or more O atoms, -C(O)O- groups and/or -C(O)NH- groups, or is a -CH₂-(cyclo hexylene)-CH₂- group;
- R²: is a hydrogen atom or a methyl group;
- R³: is a branched or preferably a linear C₁-C₁₂ alkyl group, preferably a C₁-C₁₀ alkyl group, and more preferably a C₁-C₈ alkyl group, that can be interrupted by one or O atoms, -C(O)O- groups and/or -C(O)NH- groups or that can be substituted by one or more functional groups, preferably OH, COOH and/or halogen;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms, -C(O)O- groups and/or -C(O)NH- groups;
   or
a C₅-C₂₀-alkylaryl or alkylheteroaryl group that can be interrupted by one or more O atoms, -C(O)O- groups and/or -C(O)NH- groups, and/or can be unsubstituted or substituted by one or more substituents selected from branched or preferably linear C₁-C₂₀ alkyl groups, branched or preferably linear O-C₁-C₁₂ alkoxy groups;
- R⁴: is a hydrogen atom or a methyl group;
- m: is an integer within a range from 1 to 100, preferably 2 to 50, most preferably 2 to 30;
- p: is an integer within a range from 0 to 100, preferably 2 to 50, most preferably 5 to 50;

m + p ≥ 2 and
m/(m+p) is a value within a range from 0.1 to 1.0, preferably 0.1 to 0.5, most preferably 0.2 to 0.5.

Most preferably the variables of Formula (II) have the following meanings:
- R¹: is a branched or a linear C₁-C₈ alkanediyl group, that can be interrupted by 1 to 3 O atoms and/or one -C(O)O- group, or is a -CH₂-(cyclo hexylene)-CH₂-group;
- R²: is H or preferably a methyl group;
- R³: is a branched or preferably a linear C₁-C₈ alkyl group, that can be interrupted by one O atom and/or is unsubstituted or substituted by one -OH group;
or
a C₄-C₁₆ cycloaliphatic group that can be unsubstituted or substituted by one or more substituents selected from branched or preferably linear C₁-C₆ alkyl groups, and can be interrupted by one or more O atoms, -C(O)O- groups and/or -C(O)NH- groups;
   or
a C₅-C₂₀-alkylaryl or alkylheteroaryl group that can be interrupted by one or more O atoms, -C(O)O- groups and/or -C(O)NH- groups, and/or can be unsubstituted or substituted by one or more substituents selected from branched or preferably linear C₁-C₂₀ alkyl groups, branched or preferably linear -O-C₁-C₁₂ alkoxy groups;
- R⁴: is H or preferably a methyl group;
- m: is an integer within a range from 2 to 30;
- p: is an integer within a range from 5 to 50;

m + p ≥ 2 and
m/(m+p) is a value within a range from 0.2 to 0.5.

In all instances the heteroatom of heteroaryl groups and/or alkylheteroaryl groups is preferably O, N or S and most preferably an O atom. In oligomers comprising more than one heteroaryl groups or alkylheteroaryl groups, the heteroatoms may be different or preferably the same.

Particularly preferred thiourea oligomers according to the Formula (II) are (end groups derived from transfer agents are not shown):

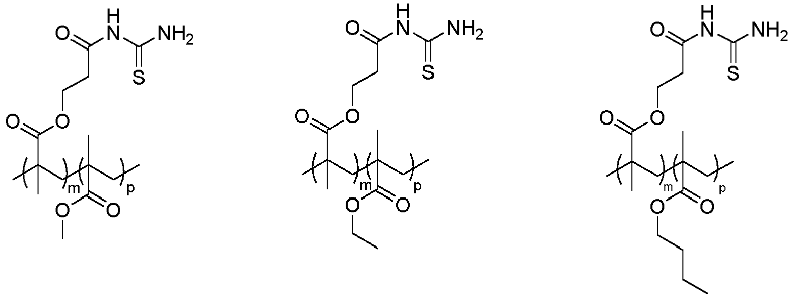

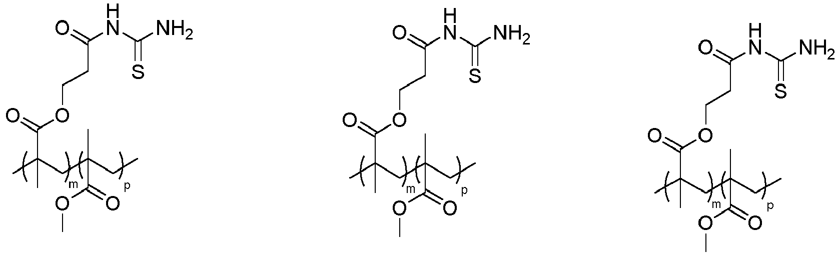

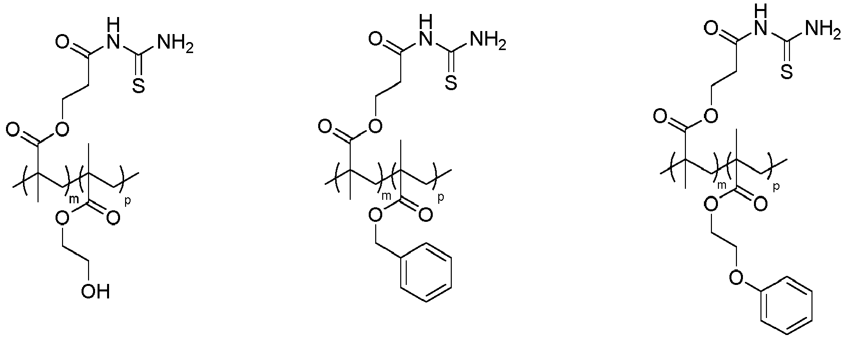

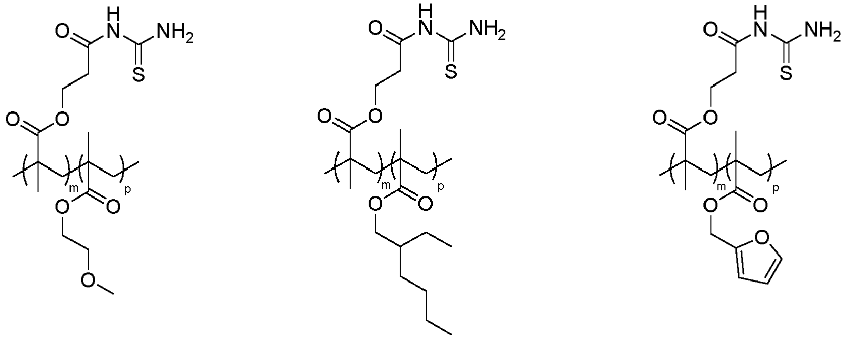

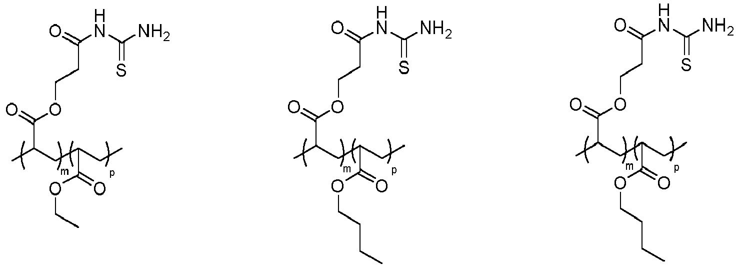

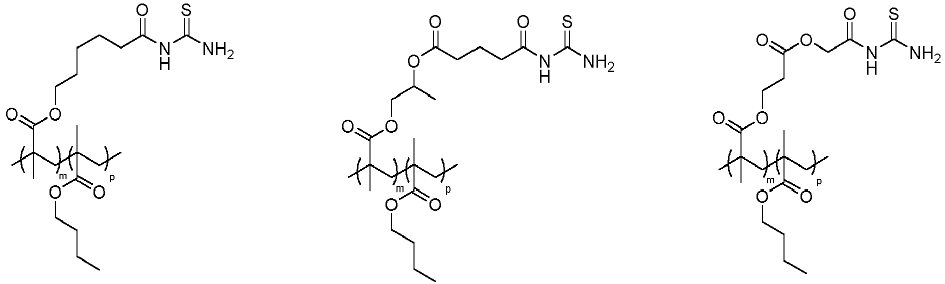

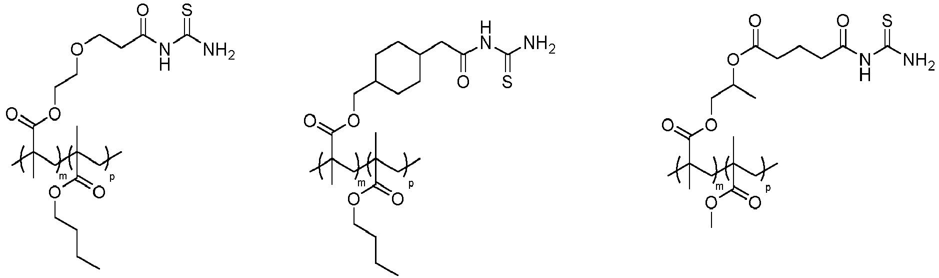

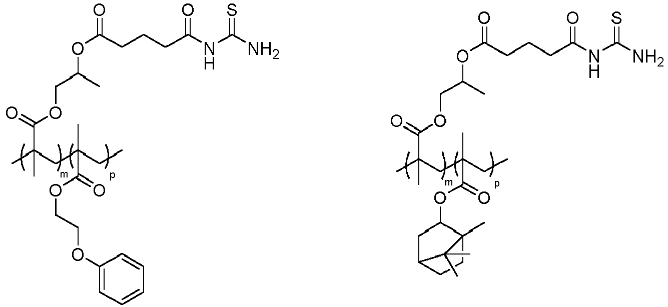

All formulae shown herein extend only to those compounds which are compatible with the theory of chemical valence. The indication that a hydrocarbon group is interrupted e.g. by one or more groups or atoms is to be understood to mean that these groups or atoms are inserted in each case into the carbon chain of the hydrocarbon group. These groups are thus bordered on both sides by C atoms and cannot be terminal. C₁ radicals cannot be interrupted. Groups can be inserted into the carbon chain in any orientation, e.g. -C(O)O- or -OC(O)-. Alkylaryl groups are groups which contain both aromatic and non-aromatic residues, such as in -CH₂-Ph or -Ph-CH₃ with Ph = phenyl. Similarly, combinations of alkanediyl groups and cycloaliphatic groups are groups comprising at least one of the above defined branched or linear alkanediyl groups and at least one of the above defined cycloaliphatic groups, such as -CH₂-(cyclo hexylene)-CH₂-.

The preferred, more preferred and most preferred definitions given for the individual variables can be selected in each case independently of each other. Compounds in which all the variables have the preferred, more preferred or most preferred definitions are naturally particularly suitable according to the invention.

The oligomers of formulas (I) and (II) are homopolymers or preferably copolymers, and the copolymers can be random copolymers or block copolymers, with random copolymers being preferred. They can be synthesized according to known synthetic routes. Preferably, a chain transfer agent (CTA) is used to control the molecular weight of the polymers.

Homopolymers are preferably synthesized via the radical polymerization of the corresponding acylthiourea (meth)acrylate monomers in the presence of a free radical initiator (e.g. AIBN).

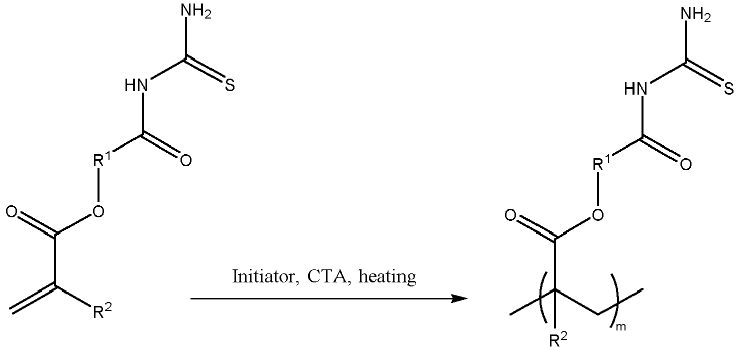

For the synthesis of copolymers, at least one, preferably only one, acylthiourea (meth)acrylate monomer is copolymerized with one or more, preferably one or two, and most preferably one comonomer. If two or more different acylthiourea (meth)acrylate monomers are used, oligomers are obtained which comprise acylthiourea (meth)acrylate units with different residues R¹ and R², for instance, R¹, R¹, R¹ and/or R², R^{2'}, R^{2"} etc. Analogously, if two or more comonomers are used, oligomers are obtained which comprise comonomer units with different residues R³, R⁴ and R⁵, for instance, R³, R^{3'}, R^{3"}, R⁴, R^{4'}, R^{4"} and/or R⁵, R⁵, R^{5"} etc.

Statistical copolymers are preferably prepared via the radical copolymerization of one acylthiourea (meth)acrylate with one or two comonomers, preferably (meth)acrylates, in the presence of a free radical initiator (e.g. AIBN) and a chain transfer agent (CTA).

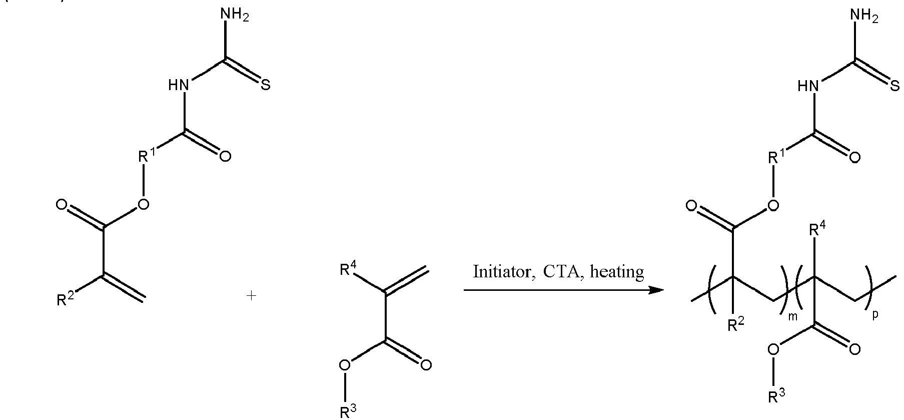

A specific example is the copolymerization of 1-(methacryloyloxy)propan-2-yl 5-oxo-5-thioureidopentanoate with methyl methacrylate (MMA) using 2-mercaptoethanol as CTA:

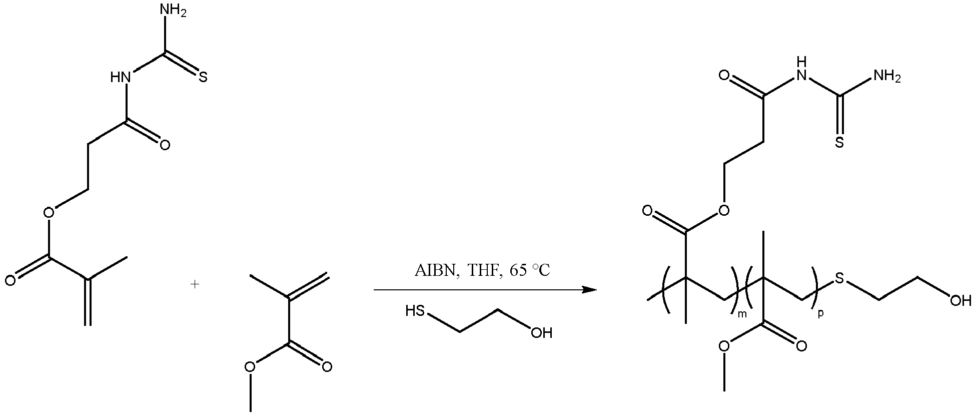

Block copolymers are preferably obtained by using the known methods of living free-radical polymerization. Preferably, the synthesis is achieved by reversible addition fragmentation chain transfer (RAFT) polymerization, atom transfer radical polymerization (ATRP) or nitroxide-mediated polymerization (NMP).

Preferred acylthiourea (meth)acrylate monomers for the synthesis of the oligomers according to Formula (I) or (II) are thiourea derivatives according to the following Formula (III): 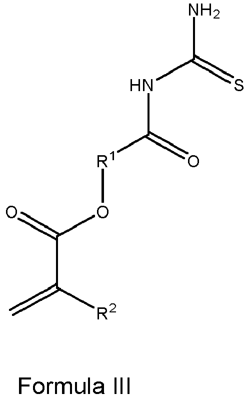 in which R¹ and R² have the meanings given above.

The acylthiourea (meth)acrylates according to Formula (III) can be prepared, for example, according to the synthetic routes that are described in EP 4 039 220 A1.

Preferred acylthiourea (meth)acrylate monomers according to Formula (III) are:

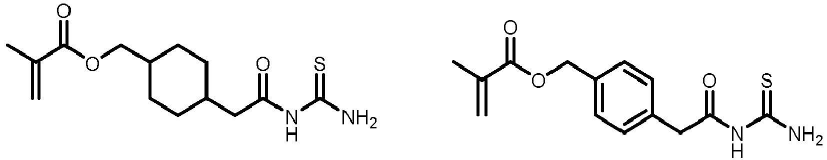

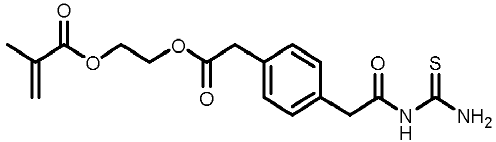

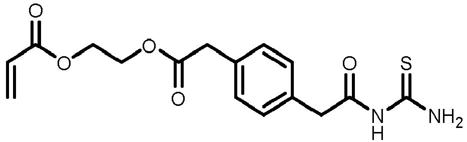

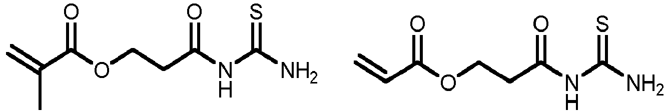

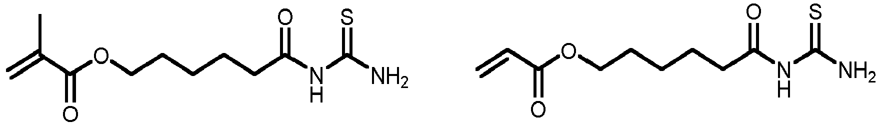

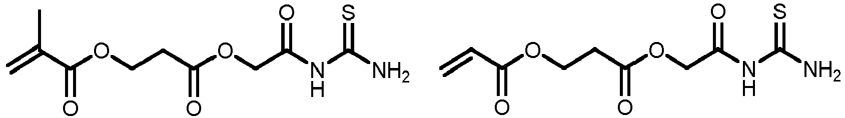

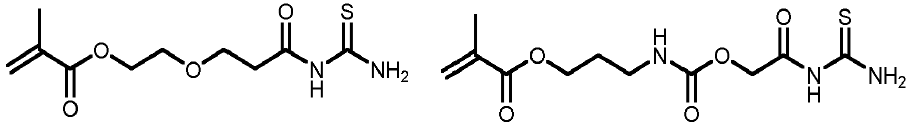

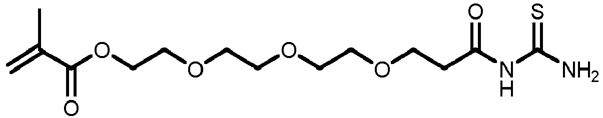

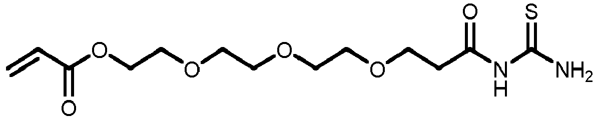

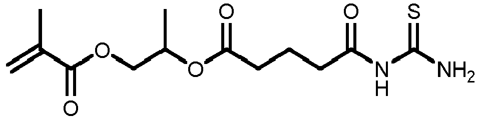

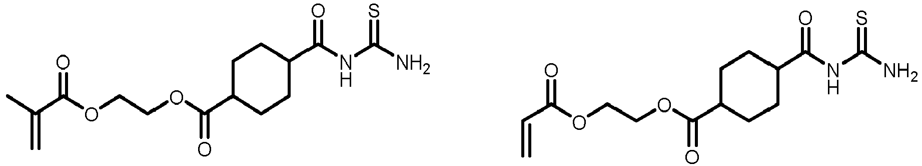

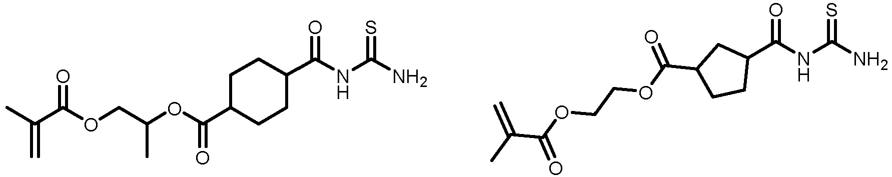

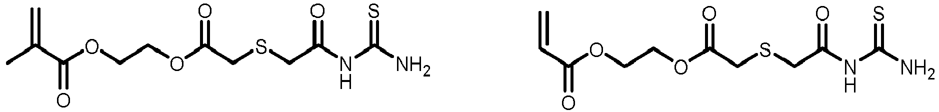

The comonomers used for the synthesis of copolymers according to Formula (I) and (II) are monofunctional monomers, preferably monofunctional (meth)acrylates. Monofunctional monomers are understood to be compounds with only one radically polymerizable group. Monofunctional monomers have no crosslinking properties and result in the formation of linear polymer chains. No multifunctional monomers, i.e., monomers with two or more radically polymerizable groups, are used.

Preferred monofunctional comonomers for the synthesis of copolymers according to Formula (I) and (II) are aromatic (meth)acrylates such as 2-phenoxyethyl (meth)acrylate, benzyl (meth)acrylate, p-cumylphenoxyethylene glycol (meth)acrylate and 2-(2-biphenyloxy)-ethyl (meth)acrylate, and cycloaliphatic monofunctional (meth)acrylates such as isobornyl (meth)acrylate, furfuryl (meth)acrylate, tetrahydrofurfuryl (meth)acrylate, dicyclopentadienyl (meth)acrylate and 4-tert-butylcyclohexyl (meth)acrylate are most preferred.

Further preferred monofunctional comonomers are (meth)acrylates comprising, in addition to the radically polymerizable group, a functional group, preferably a hydroxyl group, such as 2-hydroxyethyl (meth)acrylate, 2-hydroxypropyl (meth)acrylate as well as glycol (meth)acrylates such as ethylene glycol methyl ether (meth)acrylate, or di(ethylene glycol) methyl ether (meth)acrylate.

Furthermore, monofunctional (meth)acrylates comprising a urethane group can advantageously be used as comonomers. They can be synthesized, for example, via the reaction of a hydroxyalkyl (meth)acrylate with an isocyanate or via the reaction of an alcohol with 2-isocyanatoethyl methacrylate.

Most preferred comonomers are aliphatic linear or branched C₁-C₈ alkyl (meth)-acrylates such as methyl (meth)acrylate, ethyl (meth)acrylate, propyl (meth)acrylate, butyl (meth)acrylate, pentyl (meth)acrylate, hexyl (meth)acrylate, isopropyl methacrylate, isobutyl methacrylate and 2-ethylhexyl (meth)acrylate. Generally, methacrylates are preferred over acrylates. Styrene and vinyl acetate can also be used as comonomers.

Preferred free radical initiators for the synthesis of oligomers according to Formula (I) or (II) are azo radical initiators such as azobis(isobutyronitrile) (AIBN), 2,2'-azobis[2-methyl-N-(2-hydroxyethyl)propionamide], 1,1'-azobis(cyclohexanecarbonitrile) or 4,4'-azobis(4-cyanovaleric acid); peroxides, preferably dibenzoyl peroxide, lauroyl peroxide, tert-amyl peroxybenzoate, dicumyl peroxide, cyclohexanone peroxide, tert-butyl peroxybenzoate and di-*tert*-butyl peroxide; and peroxodisulfate s, preferably potassium peroxodisulfate K₂S₂O₈, ammonium persulfate (NH₄)₂S₂O₈ and sodium peroxodisulfate Na₂S₂O₈.

Chain transfer agents (CTA) are used to control the chain length of oligomers and polymers. Chain transfer agents are also called chain regulators. Preferred chain transfer agents are mercaptans, in particular mercaptans of the formula R"-(SH)_{z}, in which R" is a linear or branched aliphatic C₁-C₁₅ hydrocarbon radical, which can be interrupted by one or more S and/or O atoms and/or -COO- groups and can be unsubstituted or substituted by one or more -Br and/or -Cl atoms and/or -OH or -COOH groups, or an aromatic C₆-C₁₂ hydrocarbon radical, and z is 1 or 2, preferably 1. R" is preferably a branched and particularly preferably a linear C₂-C₁₅ alkyl radical, which can be substituted by functional groups, in particular -Br, -Cl, -OH and/or -COOH, and which is preferably not substituted. Particularly preferred mercaptans are lauryl mercaptan (1-dodecanethiol, DDT), 2-mercaptoethanol, 3-mercaptopropanol, 3-mer-capto-2-butanol, 2-mercapto-3-butanol, 3-mercapto-2-methyl-butan-1-ol, 3-mercapto-3-methylhexan-1-ol, 3-mercaptohexanol, 2-mercaptoacetic acid, 3-mercaptopropionic acid, 4-methylbenzenethiol, isooctyl 3-mercaptopropionate, tert-nonylmercaptan, 4,4'-thiobisbenzenethiol and 1,8-dimercapto-3,6-dioxaoctane.

Further preferred chain transfer agents are disulfides (R‴-S-S-R‴), in particular di-thiourethane disulfides, such as tetramethylthiuram disulfide and isopropylxanthic disulfide. Disulfides are also called photoiniferters because they act as photoinitiator (photoini-) during the radical photopolymerization and at the same time also take part in transfer reactions (-fer-) and termination reactions (-ter).

Moreover, silanes, in particular trimethylsilane and pentamethyldisilane, as well as halogenated compounds, in particular carbon tetrachloride, carbon tetrabromide and bromotrichloromethane, can be used as chain transfer agent.

Also preferred as chain transfer agents are addition-fragmentation chain transfer (AFCT) agents, such as vinyl ethers, allyl sulfides, allyl sulfones, allyl halides and allyl phosphonates.

The most preferred chain transfer agents according to the present invention are alkyl mercaptans according to the formula R"-SH, wherein R" is a linear C₂-C₁₅ alkyl radical, mercaptoethanol and mercaptoacetic acid.

In formulas (I) and (II), the end groups of the polymer chains, derived from the transfer agents, are not shown according to the usual conventions. When transfer agents of the formula R"-SH are used, for example, polymer chains are obtained which carry an R"-S- end group.

The acylthiourea oligomers of Formula (I) or (II) preferably have a number-average molecular weight of from 500 to 10,000 g/mol, more preferably of from 800 to 7,000 g/mol and most preferably of from 1,000 to 5,000 g/mol. According to a particularly preferred embodiment of the invention, the molecular weight is within a range of 1,500 to 4,500 g/mol. Oligomers having a molecular weight within these ranges are preferred because they are soluble in monomer mixtures without causing a major increase of viscosity. A low viscosity is advantageous because it facilitates the incorporation of inorganic fillers into the formulations, e.g. for the production of dental composites or cements. The degree of polymerization and thus the molecular weight of the acylthiourea oligomers according formulas (I) and (II) can be controlled by the amount of chain transfer agent. The degree of polymerization Pn of the oligomers is defined as the quotient of the number average polymer molecular weight Mn of the oligomers divided by the molecular weights Mu of the monomer units.

The number average molecular weight Mn is preferably determined by gel permeation chromatography (GPC). Gel permeation chromatography (GPC) is a relative method in which the molecules are separated on the basis of their size, or more precisely on the basis of their hydrodynamic volume. The absolute molecular weight is determined by calibration with known standards. Preferably, narrowly distributed polystyrene standards are used as calibration standards. These are commercially available. Styrene-divinylbenzene columns are used as separation media and tetrahydrofuran (THF) as eluent. Styrene-divinylbenzene columns are suitable for organic soluble synthetic polymers. The measurement is carried out with dilute solutions (0.05 - 0.2 wt.%) of the polymers to be investigated.

Alternatively, the number-average molecular weight can be determined by the known methods of freezing point depression (cryoscopy), boiling point elevation (ebullioscopy) or from vapor pressure depression (vapor pressure osmometry). These are absolute methods that do not require calibration standards. Concentration series of 4 to 6 diluted polymer solutions with concentrations of 0.005 to 0.10 mol/kg are examined and then the measured values are extrapolated to a concentration of 0 mol/kg.

The acylthiourea oligomers of Formula (I) or (II), in combination with a hydroperoxide compound, are active as initiators for the radical polymerization. The acylthiourea oligomers and the hydroperoxides together form a redox initiator system. The present invention also relates to a redox initiator system for the radical polymerization comprising at least one acylthiourea according Formula (I) or (II) and at least one hydroperoxide, and to radically polymerizable compositions which in addition to the at least one acylthiourea according Formula (I) or (II) and the at least one hydroperoxide comprise at least one radically polymerizable monomer. According to a further preferred embodiment, the initiator system and radically polymerizable composition additionally comprise a transition metal compound.

It is a particular advantage of the acylthiourea oligomers of Formula (I) or (II) that they are not released from the cured materials after polymerization of the polymerizable composition. They are physically and chemically bound into the polymer network. This is a significant advantage in terms of bitterness properties and possible toxic side effects. The acylthiourea oligomers of Formula (I) or (II) are therefore particularly suitable for dental and other medical applications.

The initiator systems and polymerizable compositions of the present invention contain at least one hydroperoxide compound. Preferred hydroperoxides are compounds of the formula R¹⁴-(OOH)_{y}, in which R¹⁴ is an aliphatic or aromatic hydrocarbon radical and y is 1 or 2. Preferred radicals R¹⁴ are alkyl and aryl groups. The alkyl groups can be straight-chain, branched or cyclic. Cyclic alkyl radicals can be substituted by aliphatic alkyl groups. Alkyl groups with 4 to 10 carbon atoms are preferred. Aryl groups can be unsubstituted or substituted by alkyl groups. Preferred aromatic hydrocarbon radicals are benzene radicals which are substituted with 1 or 2 alkyl groups. The aromatic hydrocarbon radicals preferably contain 6 to 12 carbon atoms. Particularly preferred hydroperoxides are t-amyl hydroperoxide, 1,1,3,3-tetramethylbutyl hydroperoxide, t-butyl hydroperoxide, t-hexyl hydroperoxide, 2,5-dimethyl-2,5-di(hydroperoxy)hexane, diisopropylbenzene monohydroperoxide, paramenthane hydroperoxide, p-isopropylcumene hydroperoxide and mixtures thereof. Cumene hydroperoxide (CHP) is quite particularly preferred.

Further preferred are the low-odor CHP derivatives of Formula IV disclosed in European patent application EP 3 692 976 A1: 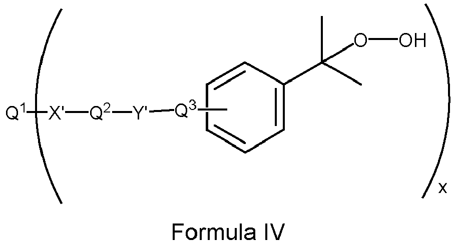 in which the variables have the following meanings:
- Q¹: a x-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR¹⁵, -Cl and -Br, wherein R¹⁵ is an aliphatic, linear or branched C₁-C₁₀ hydrocarbon radical,
- X', Y': independently of each other are in each case absent, -O-, -COO-; -CONR¹⁶-, or -O-CO-NR¹⁷-, wherein R¹⁶ and R¹⁷ independently of each other represent H or a C₁-C₅ alkyl radical, preferably H, methyl and/or ethyl, particularly preferably H, and wherein X' and Y' are preferably not absent at the same time and wherein X' and/or Y' is absent if Q² is absent,
- Q²: is absent, an aliphatic, linear or branched C₁-C₁₄ alkylene radical, which can be interrupted by S and/or O atoms and which can be unsubstituted or substituted by -OH, -OR¹⁸, -Cl and/or -Br, wherein R¹⁸ is an aliphatic, linear or branched C₁-C₁₀ hydrocarbon radical,
- Q³: a C₁-C₃ alkylene group or is absent, preferably -CH₂- or is absent,
- x: 1, 2, 3 or 4,
and wherein the substitution on the aromatic ring takes place in position 2, 3 or 4, relative to the cumene hydroperoxide group.

The variables of Formula (IV) preferably have the following meanings:
- Q¹: a mono- or divalent, aliphatic, linear or branched C₁-C₁₀ hydrocarbon radical, which can be interrupted by one or more O atoms, preferably one O atom, and which can be substituted by one or more, preferably one, substituents which are selected from -OH and -OR¹⁵ or is preferably unsubstituted, wherein R¹⁵ is an aliphatic, linear or branched C₁-C₆ hydrocarbon radical,
- X', Y': independently of each other are in each case absent, -O-, -COO- or -O-CO-NR¹⁷-, wherein R¹⁷ represents H or a C₁-C₅ alkyl radical, preferably H, methyl and/or ethyl and quite particularly preferably H, and wherein X' and Y' are preferably not absent at the same time,
- Q²: is absent, a linear or branched C₁-C₁₀ alkylene radical, which can be interrupted by one or more O atoms, preferably one O atom, and which can be substituted by one or more, preferably one, substituents which are selected from-OH and - OR¹⁸ or is preferably unsubstituted, wherein R¹⁸ is an aliphatic, linear or branched C₁-C₆ hydrocarbon radical,
- x: 1 or 2,
and wherein the substitution on the aromatic ring takes place in position 3, preferably in position 4.

More preferably the variables of Formula (IV) have the following meanings:
- Q¹: a mono- or divalent, aliphatic, linear or branched C₁-C₅ hydrocarbon radical, which can be interrupted by one O atom and which can be substituted by one OH group,
- X': -COO-,
- Y': is absent,
- Q²: is absent or a linear C₁-C₃ alkylene radical,
- x: 1 or 2,
and wherein the substitution on the aromatic ring takes place in position 4.

Most preferably the variables of Formula (IV) have the following meanings:
- Q¹: a mono- or divalent, aliphatic, branched, preferably linear C₁-C₄ hydrocarbon radical,
- X: -COO-,
- Y: is absent,
- Q²: is absent with or a methylene radical,
- x: 1 or 2, and wherein the substitution on the aromatic ring takes place in position 4.

Hydroperoxide derivatives of Formula IV particularly preferred according to the invention are:

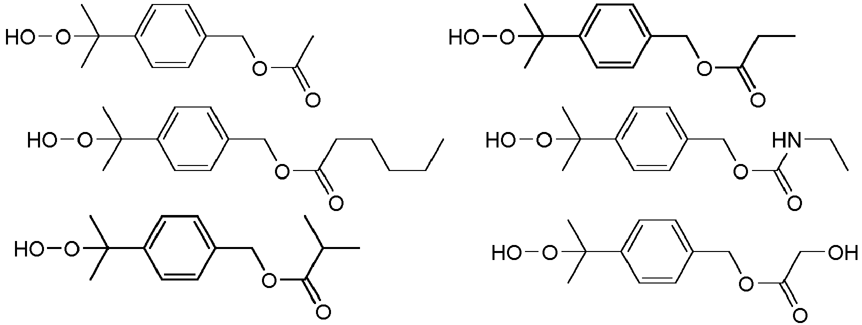

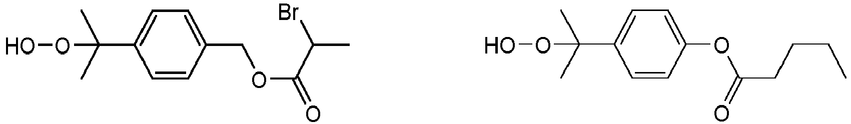

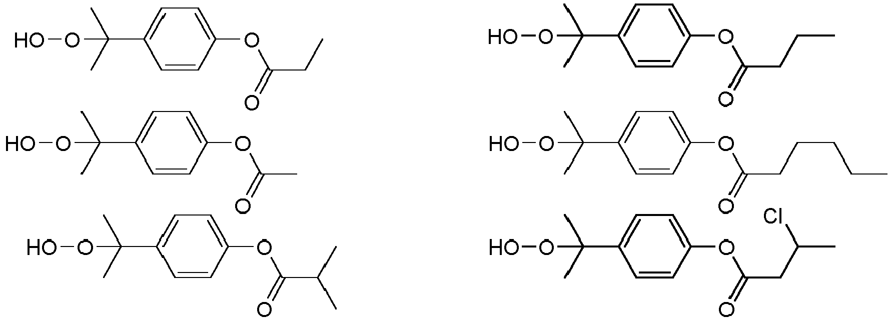

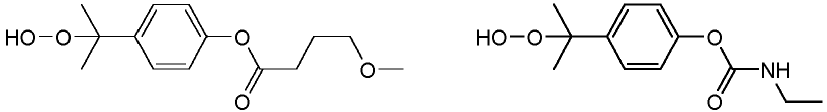

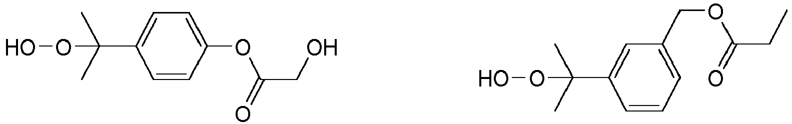

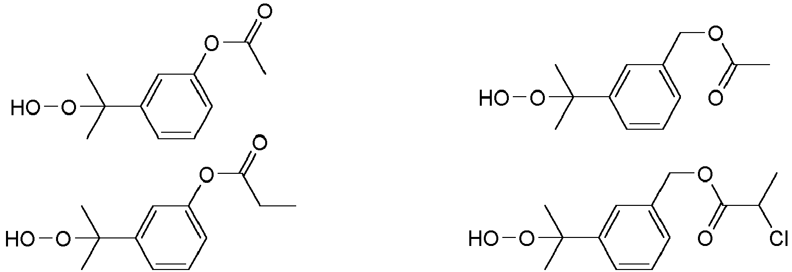

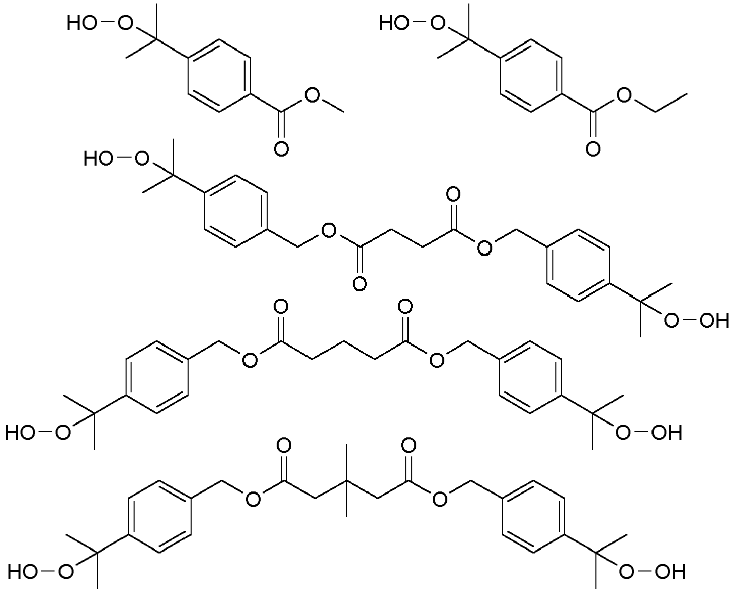

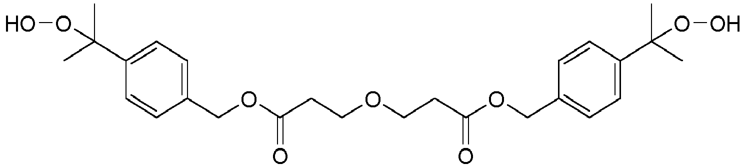

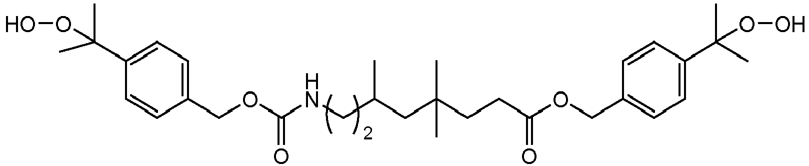

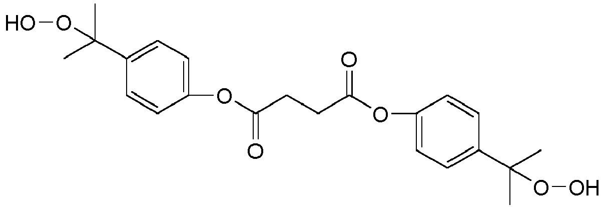

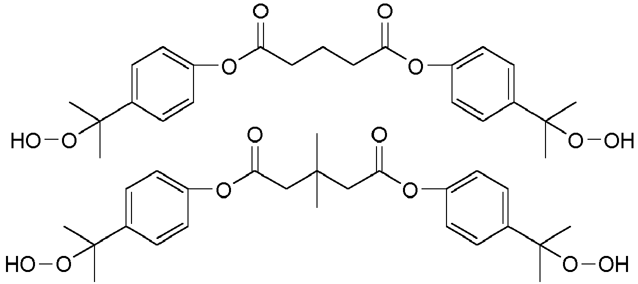

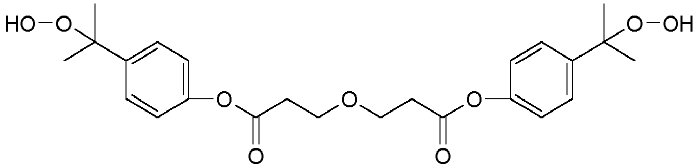

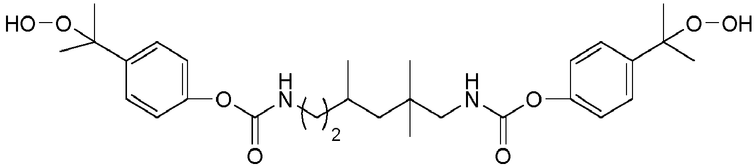

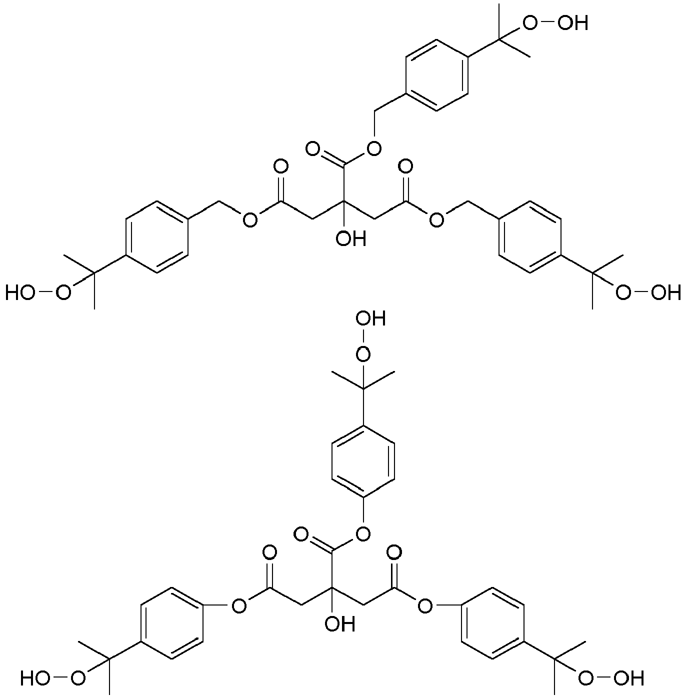

The hydroperoxide derivatives of Formula IV have a low odor and high storage stability at room temperature. They are therefore particularly suitable for the production of low-odor dental materials. The initiator systems and polymerizable compositions of the present invention can contain one or more hydroperoxides.

The initiator systems and polymerizable compositions of the present invention preferably contain at least one transition metal compound. It was found that the addition of a transition metal compound yields materials which have significantly improved mechanical properties after hardening.

Preferred transition metal compounds according to the invention are compounds which are derived from transition metals which have at least two stable oxidation states. Compounds of the elements copper, cobalt, nickel, vanadium and manganese are particularly preferred. These metals have the following stable oxidation states: Cu(I)/Cu(II), Fe(II)/Fe(III), Co(II)/Co(III), Ni(II)/Ni(III), V(III)/V(IV), V(IV)/V(V), Mn(II)/Mn(III). Materials which contain at least one copper compound are particularly preferred. In all cases, those compounds in which the respective transition metal is present in its most stable oxidation state are preferred. Compounds of Cu²⁺ are thus most preferred.

The transition metals are preferably used in the form of their salts. Preferred salts are the nitrates, acetates, 2-ethylhexanoates and halides, wherein chlorides are particularly preferred.

The transition metals can furthermore advantageously be used in complexed form, wherein complexes with chelate-forming ligands are particularly preferred. Preferred simple ligands for complexing the transition metals are 2-ethylhexanoate and THF. Preferred chelate-forming ligands are 2-(2-aminoethylamino)ethanol, aliphatic amines, particularly preferably 1,1,4,7,10,10-hexamethyltriethylenetetramine (HMTETA), N,N,N',N",N"-pentamethyldiethylenetriamine (PMDETA), tris[2-(dimethyl-amino)ethyl]amine (Me₆TREN), N,N,N',N'-tetramethylethylenediamine (TMEDA), 1,4,8,11-tetraaza-1,4,8,11-tetramethyl-cyclotetradecane (Me4CYCLAM), diethylenetriamine (DETA), triethylenetetramine (TETA) and 1,4,8,11-tetraazacyclotetradecane (CYCLAM); pyridine-containing ligands, particularly preferably N,N,N',N'-tetrakis(2-pyridylmethyl)ethylenediamine (TPEN), N, N-bis(2-pyridylmethyl)amine (BPMA), N,N-bis(2-pyridylmethyl)octylamine (BPMOA), 2,2'-bipyridine and 8-hydroxyquinoline. Most particularly preferred ligands are acetylacetone, dimethylglyoxime and 1,10-phenanthroline. Metal complexes that are soluble in the respective monomer mixture are particularly preferred.

In the case of electrically neutral ligands, the charge of the transition metal ions must be balanced by suitable counterions. For this, the above-named ions which are used to form salts are preferred wherein acetates and chlorides are particularly preferred. Chlorides and complexes are characterized by a relatively good solubility in monomers, which are used to prepare dental materials.

Instead of the transition metal complexes, non-complex salts of the transition metals in combination with complex-forming organic compounds can be used to prepare the dental materials, preferably in combination with the above-named chelate-forming compounds. The organic ligands form the catalytically active complexes when mixed with the transition metal salts. The use of such combinations of transition metal salts and organic ligands is preferred.

Preferred copper salts are CuCl, CuBr, CuCl₂, CuBr₂, CuI₂, Cu(II) carboxylates (e.g. of acetic acid or 2-ethylhexanoic acid). Preferred copper complexes are complexes with the ligands acetylacetone, phenanthroline (e.g. 1,10-phenanthroline (phen)), the aliphatic amines, such as e.g. 1,1,4,7,10,10-hexamethyltriethylenetetramine (HMTETA), N,N,N',N",N"-pentamethyldiethylenetriamine (PMDETA), tris[2-(dimethyl-amino)ethyl]amine (Me₆TREN).

Preferred iron salts are FeCl₃, FeBr₂ and FeCl₂. Preferred iron complexes are complexes with the ligands acetylacetone, triphenylphosphine, 4,4'-di(5-nonyl)-2,2'-bipyridine (dNbpy) or 1,3-diisopropyl-4,5-dimethylimidazol-2-ylidene (Prilm). The complexes Fe(acac)₂ and FeCl₂(PPh₃)₂ are quite particularly preferred.

Preferred nickel salts are NiBr₂ and NiCl₂, preferred nickel complexes are nickel acetylacetonate and NiBr₂(PPh₃)₂.

According to the invention, copper compounds, copper complexes and in particular mixtures of copper salts and complexing organic ligands are particularly preferred.

Initiators systems and polymerizable compositions which contain at least one acylthiourea oligomer of Formula (I) or (II), at least one hydroperoxide derivative and at least one transition metal compound, wherein these components are in each case selected from the above-defined preferred and particularly preferred substances, are quite particularly preferred.

The acylthiourea oligomer or oligomers of Formula (I) or (II) are preferably used in an amount of from 0.2 to 10 wt.-%, preferably 0.3 to 8.0 wt.-% and more preferably 0.5 to 5.0 wt.-%.

The hydroperoxide compound is preferably used in an amount of from 0.1 to 5.0 wt.-%, more preferably 0.2 to 4.0 wt% and most preferably 0.3 to 3.0 wt.-%.

The transition metal compound is, where applicable, preferably used in an amount of from 0.0001 to 0.5 wt.-%, more preferably 0.0005 to 0.1 wt.-% and most preferably 0.001 to 0.020 wt.-%.

Unless otherwise stated, all percentages herein relate to the total mass of the polymerizable composition.

The initiator system and the polymerizable compositions according to the invention can advantageously further comprise an initiator for the radical photopolymerization. Such compositions are dual-curing, i.e. they can be cured both chemically and by light. Photoinitiators can be added to the catalyst paste, the base paste or both.

Preferred photoinitiators are alpha-diketones derivatives, preferably camphorquinone (CQ), 9,10-phenanthrenequinone, 1-phenylpropane-1,2-dione, 2,2-dimethoxy-2-phenylacetophenone, diacetyl or 4,4'-dichlorobenzil or derivatives thereof. Camphorquinone (CQ), 2,2-dimethoxy-2-phenylacetophenone and mixtures thereof are most particularly preferred. Alpha-diketones are preferably used in combination with tertiary amines as coinitiator. Preferred coinitiators are 4-(dimethylamino)benzoic acid ester (EDMAB), N,N-dimethylaminoethyl methacrylate, N,N-dimethyl-sym.-xylidine and triethanolamine. Bimolecular photoinitiators, i.e. photoinitiators that require a second component to reach their optimal activity, are referred to as Norrish type 2 photoinitiators.

Norrish type 1 photoinitiators can also be used. The materials according to the invention preferably contain one or more Norrish type 1 photoinitiator, more preferably such a photoinitiator which is active in a wavelength range of from 400 to 500 nm.

Preferred Norrish type 1 photoinitiators are monoacyl- or bisacylphosphine oxides, diacyldialkylgermanium and tetraacylgermanium compounds as well as tetra-acylstannanes. Particularly preferred monomolecular photoinitiators are 2,4,6-trimethylbenzoyl diphenylphosphine oxide, bis(2,4,6-trimethylbenzoyl) phenylphosphine oxide, ethyl (2,4,6-trimethylbenzoyl) phenylphosphinate (TPO-L), dibenzoyldi-ethylgermane, bis(4-methoxybenzoyl)diethylgermanium (MBDEGe, trade name Ivocerin), tetrabenzoylgermane, tetrakis(o-methylbenzoyl)germane, tetrakis(mesitoyl)stannane and mixtures thereof.

Moreover, mixtures of the different photoinitiators can also be used, such as e.g. bis(4-methoxybenzoyl)diethylgermane or tetrakis(o-methylbenzoyl)germane in combination with camphorquinone and 4-dimethylaminobenzoic acid ethyl ester.

The radically polymerizable compositions of the present invention comprise at least one multifunctional (meth)acrylate and may optionally comprise one or more monofunctional (meth)acrylates. Compositions that contain at least one multifunctional (meth)acrylate and at least one monofunctional monomer are particularly preferred. The multifunctional (meth)acrylates preferably comprise 2 to 4 radically polymerizable groups.

Preferred multifunctional (meth)acrylates are bisphenol A dimethacrylate, bis-GMA (an addition product of methacrylic acid and bisphenol A diglycidyl ether), ethoxylated or propoxylated bisphenol A dimethacrylate, such as the bisphenol A dimethacrylate SR-348c (Sartomer) with 3 ethoxy groups or 2,2-bis[4-(2-methacryloyloxypropoxy)-phenyl]propane, UDMA (an addition product of 2-hydroxyethyl methacrylate and 2,2,4-trimethylhexamethylene-1,6-diisocyanate), 2-{[(2-(methacryloyloxy)ethoxy)carbonyl]amino} ethyl methacrylate, tetramethylxylylene diurethane ethylene glycol di(meth)acrylate and tetramethylxylylene diurethane ethylene glycol di(meth)acrylate, respectively, tetramethylxylylene diurethane-2-methylethylene glycoldi(meth)acrylate (V380), di-, tri- or tetraethylene glycol dimethacrylate, trimethylol propanetrimethacrylate, pentaerythritol tetramethacrylate and glycerol di- and trimethacrylate, 1,4-butanediol dimethacrylate, 1,10-decanediol dimethacrylate (D3MA), bis(methacryloyloxymethyl)tricyclo-[5.2.1.02,6]decane (DCP), polyethylene glycol or polypropylene glycol dimethacrylates, such as polyethylene glycol 200-dimethacrylate or polyethylene glycol 400-dimethacrylate (PEG-200- or PEG-400-DMA) or 1,12-dodecanediol dimethacrylate. UDMA, V-380, 2-{[(2-(methacryloyloxy)-ethoxy)carbonyl]amino} ethyl methacrylate, bisphenol A dimethacrylate SR-348c (Sartomer) and PEG-400-DMA (NK ester 9G) are particularly preferred.

The monomer V380 has the following formula:

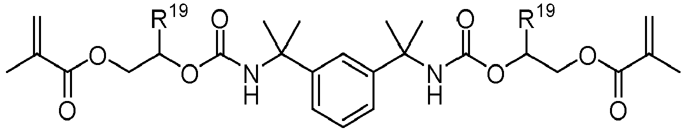

In this formula, the R¹⁹ groups are each independently H or CH₃, and the R¹⁹ residues may have the same meaning or different meanings. Preferably, a mixture is used which contains molecules in which both residues are H, molecules in which both residues are CH₃, and molecules in which one residue is H and the other residue is CH₃, the ratio of H to CH₃ preferably being 7:3. Such a mixture is obtainable, for example, by reaction of 1,3-bis(1-isocyanato-1-methylethyl)benzene with 2-hydroxypropyl methacrylate and 2-hydroxyethyl methacrylate.

Preferred monofunctional monomers are benzyl and furfuryl methacrylate, 2-phenoxyethyl methacrylate, 2-(o-biphenyloxy)ethyl methacrylate, 2-hydroxy-3-phenoxypropyl methacrylate, phenethyl methacrylate, 2-[(benzyloxycarbonyl)-amino]-ethyl methacrylate, 2-[(benzylcarbamoyl)oxy]-ethyl methacrylate, 1-phenoxypropan-2-yl methacrylate and 2-(p-cumylphenoxy)-ethyl methacrylate, tricyclodecane methacrylate, tricyclodecane methyl methacrylate and/or 2-(p-cumylphenoxy)ethyl methacrylate, and in particular cumylphenoxyethylene glycol methacrylate (CMP-1E).

According to one embodiment, the compositions according to the invention contain one or more acid-group-containing radically polymerizable monomers (adhesive monomers) in addition to the above-named monomers. These give the materials self-adhesive and/or self-etching properties. Acid-group-containing monomers are therefore particularly suitable for the preparation of self-adhesive dental materials, such as e.g. luting cements.

Preferred acid-group-containing monomers are polymerizable carboxylic acids, phosphonic acids and phosphoric acid esters as well as their anhydrides. Preferred carboxylic acids and carboxylic acid anhydrides are 4-(meth)acryloyloxyethyl trimellitic acid anhydride, 10-methacryloyloxydecylmalonic acid, N-(2-hydroxy-3-methacryl-oyloxypropyl)-N-phenylglycine, 4-vinylbenzoic acid. Preferred phosphoric acid esters are 2-methacryloyloxyethylphenyl hydrogen phosphate, 10-methacryloyloxydecyl dihydrogen phosphate (MDP) and dipentaerythritol pentamethacryloyloxyphosphate. Preferred phosphonic acids are 4-vinylbenzyl-phosphonic acid, 2-[4-(dihydroxyphos-phoryl)-2-oxa-butyl]-acrylic acid and their amides, esters, such as e.g. 2-[4-(di-hydroxyphosphoryl)-2-oxa-butyl]-acrylic acid-2,4,6-trimethylphenyl ester.

It is known that the addition of acidic monomers to two-component composites based on the hydroperoxide/thiourea redox system results in a significant reduction of the working time. Therefore, the amount of redox components is often reduced to lower the reactivity, but this leads to a deterioration of the mechanical properties. For this reason, it is challenging to formulate two-component self-adhesive cements that have both excellent mechanical properties and an adequate working time. It was surprisingly found, that the acylthiourea oligomers of Formula (I) or (II) allow the formulation of 2C materials having a favourable working time without impairing the mechanical properties.

The polymerizable compositions according to the invention can moreover advantageously contain one or more organic or inorganic fillers. Particulate fillers are preferred. Filler-containing compositions are particularly suitable as dental fixing cements or filling composites.

Preferred inorganic fillers are oxides, such as SiO₂, ZrO₂ and TiO₂ or mixed oxides of SiO₂, ZrO₂, ZnO and/or TiO₂, nanoparticulate or microfine fillers, such as pyrogenic silica or precipitated silica, glass powders, such as quartz, glass ceramic, borosilicate, or radiopaque glass powders, preferably barium or strontium aluminum silicate glasses or fluoroaluminosilicate glasses, and radiopaque fillers, such as ytterbium trifluoride, tantalum(V) oxide, barium sulfate or mixed oxides of SiO₂ with ytterbium(III) oxide or tantalum(V) oxide. The dental materials according to the invention can furthermore contain fibrous fillers, nanofibers, whiskers or mixtures thereof.

Preferably, the oxides have a particle size of from 0.010 to 15 µm, the nanoparticulate or microfine fillers preferably have a particle size of from 10 to 300 nm, the glass powders preferably have a particle size of from 0.01 to 15 µm, more preferably of from 0.2 to 1.5 µm, and the radiopaque fillers preferably have a particle size of from 0.2 to 5 µm.

Particularly preferred fillers are mixed oxides of SiO₂ and ZrO₂, with a particle size of from 10 to 300 nm, glass powders with a particle size of from 0.2 to 1.5 µm, in particular fluoroaluminosilicate glass powders and radiopaque glass powders, preferably of barium or strontium aluminium silicate glasses, and radiopaque fillers with a particle size of from 0.2 to 5 µm, in particular ytterbium trifluoride and/or mixed oxides of SiO₂ with ytterbium(III) oxide.

Preferred radiopaque glass fillers have the following composition (wt.%): SiO₂: 20-80; B₂O₃: 2-15, BaO or SrO: 10-40; Al₂O₃: 2-20; CaO and/or MgO: 0-20; Na₂O, K₂O, Cs₂O: 0-10 each; WO₃: 0-20; ZnO: 0-20; La₂O₃: 0-10; ZrO₂: 0-15; P₂O₅: 0-30; Ta₂O₅, Nb₂O₅ or Yb₂O₃: 0-5; and CaF₂ and/or SrF₂ 0-10. Particularly preferred are radiopaque glass fillers having the composition (wt.%): SiO₂: 40-75; B₂O₃: 2-15; BaO or SrO: 15-35; Al₂O₃: 2-15; CaO and/or MgO: 0-10; and Na₂O: 0-10.

Preferred fluoroaluminosilicate (FAS) glass fillers have the following composition (wt.%): SiO₂: 20-35; Al₂O₃: 15-35; BaO or SrO: 10-25; CaO: 0-20; ZnO: 0-15; P₂O₅: 5-20; Na₂O, K₂O, Cs₂O: 0-10 each; and CaF₂: 0.5-20. Particularly preferred are FAS fillers with the composition (wt.%): SiO₂: 20-30; Al₂O₃: 20-30; BaO or SrO: 10-25; CaO: 5-20; P₂O₅: 5-20; Na₂O: 0-10; and CaF₂: 5-20.

The FAS fillers and radiopaque glass fillers preferably have an average particle size of 0.2 to 20 µm and particularly preferably of 0.4 to 5 µm.

According to a preferred embodiment of the invention, the fluoroaluminosilicate glass filler and/or radiopaque glass filler has been pre-treated with acid. By the acid treatment of the filler, the storage stability of two-component materials containing acidic monomers can be significantly improved. Preferred acid treated fillers and their preparation are described in European Patent Application No. 21218194.5, which is incorporated herein by reference.

The acid treatment of the particulate FAS or glass fillers is carried out by washing the filler with acid, preferably by the following process:
(i) The particulate FAS or glass filler is dispersed in an aqueous solution of an organic or preferably inorganic acid. The acid is preferably used in a concentration of 0.1 to 5 mol/l, particularly preferably 0.5 to 3 mol/l.
(ii) The dispersion from step (i) is then stirred, preferably for 0.5 to 24 h, more preferably for 1 to 5 h.
(iii) After the stirring in an acid solution, the filler is separated and washed with deionized water. For this purpose, it is preferably dispersed in water and stirred for 1 to 60 minutes, particularly preferably for 2 to 20 minutes.
(iv) After washing, the filler is separated and dried, preferably in a vacuum drying oven in a fine vacuum at 20 to 80 °C, particularly preferably at 40 to 60 °C. The filler is preferably dried to constant weight.
(v) After drying, the filler is preferably subjected to an optional thermal treatment. For this purpose, it is preferably heated for 2 to 12 h, particularly preferably for 4 to 6 h, to a temperature that is well below the glass transition temperature of the filler, preferably 200 to 500 °C and particularly preferably 300 to 400 °C.

The acid used to treat the fillers is preferably completely removed from the fillers after the acid treatment. The fillers are not coated with an acid.

The acid treatment can be repeated once or several times. Steps (i) and (iii) are preferably carried out at a temperature in the range from 5 to 50°C, particularly preferably at room temperature (23°C). The temperature is measured in the solution or dispersion in each case.

Acids that form soluble salts with Ca, Al, Sr and Ba ions are preferred. Particularly preferred are formic acid, acetic acid and especially hydrochloric acid, and nitric acid. Alternatively, acids that form sparingly soluble salts with Ca, Al, Sr or Ba ions, such as phosphoric acid, may be used but these are less preferred. Poorly soluble salts are defined as salts with a solubility of less than 0.1 g/l (in water at room temperature). According to the invention, acidic organic monomers and acidic organic polymers as well as peracids and hydrofluoric acid are not suitable as acids.

The washing step (iii) is preferably repeated 1 to 5 times, particularly preferably 3 times, so that the acid is completely removed from the filler. For this purpose, the filler is separated from the water following step (iii) and again dispersed and stirred in deionized water. The washing is repeated until the pH of the water in the last washing step is ≥ 5. After washing, the filler can be subjected to further acid treatment and washing. The sequence of acid treatment and washing can be repeated once or several times.

The acid treatment of the fillers significantly improves the storage stability of the compositions according to the invention. In particular, the content of radical polymerizable monomers comprising an acid group decreases only slowly, so that the compositions show high adhesion to the tooth structure and particularly to dentin even after prolonged storage. The acid treatment thus significantly improves the properties of dental composites with self-adhesive properties in a simple manner.

According to another preferred embodiment of the invention, the fluoroaluminosilicate glass fillers and/or radiopaque glass fillers are preferably used in combination with at least one sulfate and/or phosphate which is water-soluble at 20°C. Water-soluble sulfates or phosphates were found to improve the storage stability of compositions comprising a fluoroaluminosilicate glass filler and/or radiopaque glass filler in combination with acidic monomers. The fluoroaluminosilicate glass filler and/or radiopaque glass filler may be used in acid-treated or non-acid-treated form. Preferred fillers comprising a fluoroaluminosilicate glass and/or radiopaque glass in combination with at least one water-soluble sulfate and/or phosphate and their preparation are described in European Patent Application No. 21218209.1, which is incorporated herein by reference.

Preferred sulfates are inorganic salts of sulfuric acid and preferred phosphates are inorganic salts of orthophosphoric acid (H₃PO₄). Water-soluble sulfates or phosphates are sulfates or phosphates which preferably have a water solubility of at least 100 g/l, preferably of 110 to 1,000 g/l and particularly preferably of 150 to 800 g/l. Preferred water-soluble sulfates are potassium sulfate (K₂SO₄; water solubility 111 g/l), sodium sulfate (Na₂SO₄; water solubility 170 g/l) and particularly preferably ammonium sulfate ((NH₄)₂SO₄; water solubility 754 g/l). Preferred water-soluble phosphates are potassium phosphate (K₃PO₄; water solubility 508 g/l), sodium phosphate (Na₃PO₄; water solubility 285 g/l) and especially preferably ammonium phosphate ((NH₄)₃PO₄; water solubility 580 g/l). All solubility data refer to solubility in water at 20°C.

The one or more water-soluble sulfates and/or water-soluble phosphates are preferably added in a total amount of at least 0.3 wt.-% and, more preferably, at least 0.4 wt.-%. According to the invention, compositions are preferred which contain up to 0.3 to 9.0 wt.-%, more preferably 0.4 to 6.0 wt.%, and most preferably from 0.7 to 4.0 wt.-% of at least one water-soluble sulfate and/or phosphate. Unless otherwise stated, all percentages herein are based on the total mass of the composition. The sulfates or phosphates may be present in dissolved or preferably in solid form. According to the invention, sulfates are preferred.

According to still another preferred embodiment of the invention, the fluoroaluminosilicate glass filler (FAS filler) and/or radiopaque glass filler is coated with a silica (hetero)polycondensate. Preferred coated fillers and their preparation are described in European Patent Application No. 22202860.7, which is incorporated herein by reference. The term silica (hetero)polycondensate stands for silica polycondensate or silica hetero polycondensate. Surprisingly, it was found that coating the filler with a silica (hetero)polycondensate results in a significant increase in the storage stability of the compositions without deteriorating the other properties. The coating of the fillers prevents significant amounts of metal ions, in particular Al, Ca, Ba or Sr ions, from diffusing from dental fillers, such as radiopaque glass fillers and FAS fillers, into the resin mixture and reacting there with acidic monomers. It also prevents acidic monomers from binding to the filler surface by forming insoluble salts. This avoids a decrease in the concentration of acid monomers in the resin matrix and a consequent deterioration in adhesion, and improves the storage stability of the materials.

The filler is preferably coated by a sol-gel process. For this purpose, a sol is prepared by hydrolytic condensation of one or more condensable compounds of silicon and optionally other elements from the group boron, aluminum, titanium or phosphorus and/or precondensates derived from these compounds, brought into contact with the filler and the filler is subsequently dried.

Preferred hydrolytically condensable compounds of silicon are silanes of the general Formula (V):

SiX"ₐR'₄₋ₐ Formula V

in which the variables have the following meanings:
- R' =: C₁-C₂₀-alkyl or C₂-C₁₀-alkenyl, where alkyl and alkenyl radicals may comprise one or more O or S atoms, ester, amide, amino or urethane groups, C₆-C₁₄-aryl or C₇ -C₁₅-alkylaryl;
- X" =: halogen, hydroxy, C₁-C₁₀-alkoxy, C₁-C₆-acyloxy;
- a =: 2, 3 or 4, preferably 3 or 4.

If there are multiple R' or X" residues, they may be different or preferably the same. Preferred alkylaryl groups are -CH₂ -CH₂ -Ph or a -Ph-CH₃ group with Ph = phenyl.

Particularly preferred are silanes of the Formula (V) in which at least one and preferably all variables have one of the following meanings:
- R' =: C₁-C₁₀-alkyl or C₂-C₈-alkenyl, where alkyl and alkenyl radicals may comprise one or more O atoms or ester groups, phenyl or C₇-C₁₅-alkylaryl-,
- X" =: hydroxy, C₁-C₅-alkoxy-, C₁-C₃-acyloxy;
- a =: 3 or 4.

The silanes of Formula (V) can be hydrolytically condensed via the radicals X" to form an inorganic network with Si-O-Si units. If the radicals R' contain radically polymerizable groups, an organic network can also be formed via them.

More preferred are silanes of the Formula (V) where the variables have the following meanings:
- R': methyl, ethyl, n-propyl, i-propyl, n-butyl, s-butyl, n-pentyl, vinyl, a (meth)acryl group (H₂C=C(CH₃ )-COO-), allyl, phenyl or naphthyl, particularly preferred are ethyl, n-propyl, i-propyl and most preferred are methyl or 3-(meth)-acryloyloxypropyl;
- X": methoxy, ethoxy, n-propoxy, i-propoxy, n-butoxy, F, Cl, Br, especially preferably Cl and most preferably ethoxy;
- a: 3 or 4.

According to the invention, silanes of the formula SiX"₄ (a = 4) are particularly preferred, with Si(OCH₃)₄ and Si(OC₂H₅)₄ being even more preferred. Silanes of the formula SiX"₄ give a particularly high network density. They are preferably used in an amount of 40 to 100% by weight, more preferably 50 to 100% by weight and most preferably 60 to 95% by weight, based on the total amount of silanes of Formula (V).

Further preferred are silanes of Formula (V) containing at least one free-radically polymerizable group, 3-methacryloyloxypropyltrimethoxysilane being particularly preferred. The radically polymerizable group enables covalent bonding between the coated fillers and the radically polymerizable matrix during curing of the materials according to the invention. Silanes containing radically polymerizable groups are preferably used in an amount of 0 to 10% by weight, more preferably 0 to 8% by weight and most preferably 1 to 5% by weight, based on the total amount of silanes of the Formula (V).

In addition to the silane(s) of Formula (V), one or more further hydrolytically condensable compounds of the elements Al, Ti, B or P can be used to coat the fillers. Preferred compounds are those of Al, Ti and B. Preferred aluminum compounds are aluminum sec-butylate and aluminum isopropylate. Preferred titanium compounds are Ti(OC₂H₅)₄, Ti(OC₃H₇)₄, Ti(O-iC₃H₇)₄ and Ti(OC₄H₉)₄. Preferred boron compounds are B(OCH₃)₃ and B(OC₂H₅)₃ . By using additional hydrolytically condensable compounds, the properties of the coating, such as hardness and abrasion resistance, can be specifically controlled. One or more additional compounds may be used. These are preferably used in a total amount of 0 to 20% by weight, more preferably 0 to 15% by weight and most preferably 0 to 10% by weight, based on the total amount of silanes of Formula (V). Metal alkoxides, for example of Al or Ti, can also be used in complexed form, for example in the form of the reaction products of Al or Ti alkoxides with (meth)acrylic acid or 2-acetoacetoxyethyl methacrylate. The proportion of silanes of the Formula (V) is preferably 80 to 100% by weight, more preferably 85 to 100% by weight and most preferably 90 to 100% by weight, based on the total amount of hydrolytically condensable compounds.

The silane or silanes of Formula (V) and optionally other hydrolytically condensable compounds are hydrolyzed and condensed in the presence of water, preferably at least the amount of water stoichiometrically required for hydrolysis, and optionally a condensation catalyst and/or a solvent. Polycondensates or heteropolycondensates are formed in this process. Instead of the hydrolytically condensable compounds, precondensates derived therefrom can also be used. Precondensates are reaction products of the alkoxysilanes and/or halosilanes and optionally of the metal alkoxides with water with partial or complete elimination of the alcohols or hydrogen halides. In the case of the alkoxysilanes, silanols are formed as precondensates.

Preferably, the hydrolytic condensation is carried out by dispersing the filler to be coated in the silane(s) of Formula (V) and, if desired, further hydrolytically condensable compounds or, preferably, a solution of the silane(s) and, if desired, further hydrolytically condensable compounds in a suitable solvent and then adding water. The mixture is preferably stirred in this process.

If only silanes are used, the hydrolysis is preferably carried out at room temperature or under slight cooling. Preferably, a hydrolysis or condensation catalyst is added. Polycondensates are obtained in this process. The resulting mixture (dispersion) is preferably stirred for several hours to several days at a temperature of -20 to 150°C, preferably 20 to 110°C. The filler is then separated, e.g. by centrifugation, then optionally washed with a solvent one or more times and then dried. Drying can be carried out at room temperature or at elevated temperature, preferably at 50 to 60°C. Preferably, the filler is dried in a vacuum drying cabinet. Drying may extend over a period of several hours to several days. Preferably, drying is carried out until the weight is constant. After drying, the fillers may be subjected to further heat treatment. This is preferably carried out at a temperature of from 20 to 150°C, more preferably from 30 to 100°C and most preferably from 40 to 80°C. Temperature and treatment time are selected so that any organic groups present in the coating are not destroyed and no glass layer is formed.

If, in addition to the silanes of Formula (V), other hydrolytically condensable compounds are used to coat the filler, the water is preferably added in stages at -20 to 100°C, preferably at 0 to 30°C. In this case, heteropolycondensates are formed. The method of water addition depends primarily on the reactivity of the hydrolytically condensable compounds used. The desired amount of water can be added, for example, in portions or in one portion.

In the preparation of both polycondensates and heteropolycondensates, the water is preferably added in an amount of 0.5 to 10 moles of water per mole of hydrolyzable groups, particularly preferably in an amount of 1 to 10 moles of water per mole of hydrolyzable groups and most preferably in an amount of 1.5 to 5 moles of water per mole of hydrolyzable groups of the silanes of Formula (V) and optional other hydrolytically condensable compounds. The water or portions thereof may be introduced in pure form or as a constituent of other components, e.g. of the catalyst solution.

Preferred solvents for performing the hydrolytic condensation are lower aliphatic alcohols, such as ethanol or i-propanol, lower dialkyl ketones, such as acetone or methyl isobutyl ketone, cyclic ethers, such as tetrahydrofuran or dioxane, aliphatic ethers, such as isopropoxyethanol or isopropoxypropanol, amides, such as dimethylformamide, and mixtures thereof.

Preferred hydrolysis or condensation catalysts are organic or inorganic acids, such as hydrochloric acid, sulfuric acid, p-toluenesulfonic acid, formic acid or acetic acid. Ammonium fluoride and organic bases, in particular amines, such as n-propylamine, diethylamine or triethylamine, and aminosilanes, such as 3-aminopropyltrimethoxysilane, are further preferred.

The condensation time depends on the respective starting components, their proportions, the catalyst used, if any, the reaction temperature, etc. Preferably, the reaction time is in the range of 1 to 96 h, more preferably 2 to 72 h and most preferably 3 to 48 h.

When drying the fillers and evaporating the solvent, the hydrolysis and condensation reaction leads to the formation of a gel film via the formation of sol particles, which further cross-links in the course of drying and forms a dense inorganic network on the filler surface. The properties of the coating, such as strength, flexibility, adhesion, hardness, refractive index and impermeability, can be specifically adjusted by the selection of the silanes and the other hydrolytically condensable components and adapted to the particular application. For example, the functionality of the silanes of Formula (V), which can vary between 4, e.g. in tetraethoxysilane (TEOS), and 2, e.g. in dialkoxysilane, can be used to adjust the degree of crosslink-linking and thus the strength, flexibility and impermeability of the coating. By selecting the organic groups of the silanes, it is possible to specifically influence, for example, the flexibility, polarity, wetting or thickening effect of the coating. Silanes with polymerizable groups, such as in 3-methacryloyloxypropyltrimethoxysilane, allow additional crosslinking of the coating by radical polymerization and covalent incorporation of the fillers into the polymer matrix. Co-condensation with metal alkoxides, such as Ti alkoxide, allows control of the kinetics of the hydrolytic condensation and, for example, an increase in density and hardness as well as adjustment of the refractive index of the coating.

The filler is preferably dispersed in the hydrolysis mixture in an amount of from 1 to 25% by weight, more preferably from 3 to 20% by weight and most preferably from 4 to 15% by weight, based on the total mass of the mixture. According to the invention, particulate fillers are preferred.

Furthermore, so-called inert fillers can also be used as fillers. These are glass fillers whose surface is coated with a diffusion barrier layer, e.g. on a sol-gel basis, or with a polymer layer, e.g. of PVC. Preferred fillers are those described in EP 2 103 296 A1.

Polymer particles, in particular particles of crosslinked organic polymers, are preferred as organic fillers. Polymer particles can be obtained by curing one or more monomers and then grinding the cured mixture, or by suspension polymerization. The mixtures can be cured by light irradiation or thermally. Radically polymerizable monomers and in particular the monomers listed above are preferred as monomers. Dimethacrylates, specifically bis-GMA, UDMA, TMX-UDMA, DCP, D3MA and mixtures thereof, are most particularly preferred.

Polymer particles prepared by grinding exhibit a splintery appearance under the microscope and are therefore also referred to as splinter polymerizates or splinter polymers. Pearl polymerizates obtained by suspension polymerization in contrast are characterized by spherical particles. Splinter polymerizates are preferred as organic fillers.

In addition, so-called composite fillers, i.e. organic-inorganic fillers, are preferred as fillers. These are also referred to as isofillers. These are fragmentary polymers which in turn contain an inorganic filler, preferably pyrogenic SiO₂ and/or ytterbium trifluoride. Preferred are polymers based on dimethacrylates. For the production of isofillers, the inorganic filler(s) is/are incorporated, for example, into a dimethacrylate resin matrix, and the resulting composite paste is subsequently thermally polymerized and then ground.

A composite filler preferred according to the invention can be prepared, for example, by thermally curing a mixture of Bis-GMA (8.80% by weight), UDMA (6.60% by weight), 1,10-decanediol dimethacrylate (5.93% by weight), dibenzoyl peroxide + 2,6-di-tert. butyl-4-methylphenol (together 0.67 wt.%), glass filler (average grain size 0.4 µm; 53.0 wt.%) and YbF₃ (25.0 wt.%) and subsequent grinding of the cured material to the desired grain size. Another composite filler preferred according to the invention is a hot-cured composite filler ground to the desired grain size and prepared from UDMA (23.58 % by weight), 1,10-decanediol dimethacrylate (5.92 % by weight), dibenzoyl peroxide (0.50 % by weight), fumed silica Aerosil OX50 (50.0 % by weight) and YbF₃ (20.0 % by weight). All percentages refer to the total mass of the composite filler.

Unless otherwise stated, all particle sizes are weight-average particle sizes, wherein particle-sizes within the range of from 0.1 µm to 10 µm are measured by means of static light scattering, preferably using an LA-960 static laser scattering particle size analyzer (Horiba, Japan). Here, a laser diode with a wavelength of 655 nm and an LED with a wavelength of 405 nm are used as light sources. The use of two light sources with different wavelengths makes it possible to measure the entire particle-size distribution of a sample in only one measurement pass, wherein the measurement is carried out as a wet measurement. For this, a 0.1 to 0.5% aqueous dispersion of the filler is prepared, and the scattered light thereof is measured in a flow cell. The scattered-light analysis for calculating particle size and particle-size distribution is performed in accordance with the Mie theory according to DIN/ISO 13320: 2020.

Particle sizes smaller than 0.1 µm are preferably determined by means of dynamic light scattering (DLS). The measurement of the particle size in the range of from 5 nm to 0.1 µm is preferably effected by dynamic light scattering (DLS) of aqueous particle dispersions, preferably with a Malvern Zetasizer Nano ZS (Malvern Instruments, Malvern UK) with a He-Ne laser with a wavelength of 633 nm, at a scattering angle of 90° at 25° C.

Particle sizes smaller than 0.1 µm can also be determined by means of SEM or TEM spectroscopy. The transmission electron microscopy (TEM) is preferably carried out with a Philips CM30 TEM at an accelerating voltage of 300 kV. For the preparation of the samples, drops of the particle dispersion are applied to a 50 Å thick copper grid (mesh size 300), which is coated with carbon, and then the solvent is evaporated.

The light scattering decreases as the particle size decreases, but fillers with a small particle size have a greater thickening action. The fillers are divided according to their particle size into macrofillers and microfillers, wherein fillers with an average particle size of from 0.2 to 10 µm are called macrofillers and fillers with an average particle size of from approx. 5 to 100 nm are called microfillers. Macrofillers are obtained e.g. by grinding e.g. quartz, radiopaque glasses, borosilicates glasses or glass ceramics and usually consist of splintery parts. Microfillers such as mixed oxides can be prepared e.g. by hydrolytic co-condensation of metal alkoxides.

To improve the bond between the filler particles and the crosslinked polymerization matrix, the fillers are preferably surface-modified, particularly preferably by silanization, most particularly preferably by radically polymerizable silanes, in particular with 3-methacryloyloxypropyltrimethoxysilane. For the surface-modification of non-silicate fillers, e.g. of ZrO₂ or TiO₂, functionalized acidic phosphates, such as e.g. 10-methacryloyloxydecyl dihydrogen phosphate can also be used.

The compositions according to the invention preferably contain from 20% to 90% by weight, more preferably from 40% to 85% by weight and most preferably from 50% to 80% by weight of fillers, in each case based on the total mass of the composition.

The compositions according to the invention may also contain further additives, in particular stabilizers, such as e.g. polymerization stabilizers, dyes, microbiocidal active ingredients, fluoride-ion-releasing additives such as fluoride salts in particular NaF, ammonium fluoride, or fluorosilanes, optical brighteners, fluorescent agents, plasticizers, and/or UV absorbers.

The compositions according to the invention are particularly suitable as dental materials, in particular as luting materials, filling composites and temporary filling materials.

Compositions which comprise:
(a) 0.2 to 10.0 wt.-%, preferably 0.3 to 8.0 wt.-% and more preferably 0.5 to 5.0 wt.-% of at least one acylthiourea oligomer according to Formula (I) or preferably Formula (II),
(b) 0.1 to 5.0 wt.-%, preferably 0.1 to 4.0 wt.-% and more preferably 0.2 to 2.0 wt.-% of at least one hydroperoxide compound,
(c) 5 to 80 wt.-%, preferably 10 to 60 wt.-% and more preferably 15 to 50 wt.-% of at least one radically polymerizable monomer,
(d) 20 to 90 wt.-%, preferably 40 to 85 wt.-% and more preferably 50 to 80 wt.-% filler(s), and
(e) 0.001 to 5 wt.-%, preferably 0.01 to 3 wt.-%, additive(s),
are preferred according to the invention for the use as dental material.

All quantities herein are relative to the total mass of the composition, unless otherwise stated.

The filling level depends on the desired intended use of the composition. Filling composites preferably have a filler content of from 50 to 85 wt.-% and luting composites of from 10 to 70 wt.-%.

Compositions which additionally contain
(f) 0.0001 to 0.5 wt.-%, preferably 0.0005 to 0.1 wt.-% and particularly preferably 0.001 to 0.02 wt.-% of at least one transition metal compound
are particularly preferred.

Compositions comprising at least one acylthiourea oligomer according to Formula (I) or (II) and at least one hydroperoxide are self-curing. They are used in the form of two spatially separated components, i.e. as a 2-component system (2C system). Oxidizing and reducing agents are incorporated into separate components of the composition. One component, the so-called catalyst paste, contains the hydroperoxide, and optionally a photoinitiator, and the second component, the so-called base paste, contains the acylthiourea oligomer, optionally a photoinitiator and optionally catalytic amounts of a transition metal compound. Polymerization is initiated by mixing the components. Compositions containing both a redox initiator and a photoinitiator are referred to as dual-curing. The pastes are mixed together shortly before use, preferably with a double-push syringe. Base paste and catalyst paste are preferably mixed with each other in a volume ratio of 1:1. The compositions specified above relate to the mixed pastes.

The compositions according to the present invention preferably comprise a catalyst paste comprising:
(b) 0.2 to 10.0 wt.-%, preferably 0.2 to 8.0 wt.-% and more preferably 0.4 to 4.0 wt.-% of at least one hydroperoxide compound,
(c) 5 to 80 wt.-%, preferably 10 to 60 wt.-% and more preferably 15 to 50 wt.-% of at least one radically polymerizable monomer,
(d) 20 to 90 wt.-%, preferably 40 to 85 wt.-% and more preferably 50 to 80 wt.-% filler(s), and
(e) 0.001 to 5 wt.-%, preferably 0.01 to 3 wt.-%, additive(s),

in each case relative to the total mass of the catalyst paste, and
a base paste comprising:
   (a) 0.4 to 20.0 wt.-%, preferably 0.6 to 16.0 wt.-% and more preferably 1.0 to 10.0 wt.-% of at least one acylthiourea oligomer according to Formula (I) or preferably Formula (II),
   (c) 5 to 80 wt.-%, preferably 10 to 60 wt.-% and more preferably 15 to 50 wt.-% of at least one radically polymerizable monomer,
   (d) 20 to 90 wt.-%, preferably 40 to 85 wt.-% and more preferably 50 to 80 wt.-% filler(s),
   (e) 0.001 to 5 wt.-%, preferably 0.01 to 3 wt.-%, additive(s), and
   (f) 0.0002 to 1.0 wt.-%, preferably 0.0010 to 0.2 wt.-% and particularly preferably 0.002 to 0.04 wt.-% of at least one transition metal compound,
in each case relative to the total mass of the base paste.

The compositions according to the invention are particularly suitable as dental materials for intraoral use by the dentist for the restoration of damaged teeth (therapeutic use), especially as temporary materials, filling composites and luting materials.

Two-component composites according to the invention can be used advantageously for the direct filling therapy. In the case of caries, the dentist first removes the carious tissues, e.g., with a rose-head burr. After the optional application of a dental adhesive, a 2C composite according to the invention is placed in the cavity. Due to the redox polymerization, a high depth of cure can be achieved. According to a preferred embodiment of the invention, the composite material additionally comprises a photoinitiator, and an additional photopolymerization step is performed. In the case of self-adhesive two-component composites, the material can be applied directly to the tooth without the need of a dental adhesive.

The compositions according to the invention can also be used for the fabrication of high-quality temporaries for dental crowns and bridges as well as for temporary inlays, onlays and veneers directly at chairside.

Furthermore, the compositions according to the invention can also be used for the cementation of indirect restorations such as crowns, bridges, inlays, onlays or veneers. In this case, the described compositions are used to mediate a strong bond between the tooth substance and the indirect restoration, e.g., a ceramic restoration or a cured composite. They can be used as luting composites or self-adhesive resin cements.

The invention is explained in more detail below with reference to examples.

### Examples

### Example 1

### Synthesis of 1-(methacrylovloxy)propan-2-yl 5-oxo-5-thioureidopentanoate TU1

### Step 1: Synthesis of 5-[(1-methacryloyloxypropan-2-yl)oxy]-5-oxopentanoic acid

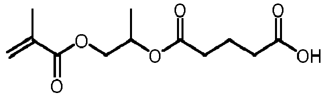

A solution of triethylamine (48.63 g, 0.48 mol), 2-hydroxypropyl methacrylate (69.28 g, 0.48 mol), and 4-dimethylaminopyridine (0.29 g, 2.4 mmol) in dichloromethane (150 mL) was slowly added to a solution of glutaric anhydride (54.83 g, 0.48 mol) in dichloromethane (300 mL) at 0°C. The reaction mixture was stirred at 0°C for 2h and at ambient temperature for 18 h. The reaction mixture was washed with hydrochloric acid (2 *N,* 3× 200 mL), water (2 × 200 mL), and brine (200 mL), dried over anhydrous magnesium sulfate, filtered, and the solvent was evaporated. The residue was dissolved in ethyl acetate (500 mL) and cooled to 0°C. Cold saturated aqueous Na₂CO₃ solution (400 mL) was added while stirring. The phases were separated and the aqueous phase was washed with ethyl acetate (3x 150mL). The aqueous phase was acidified with hydrochloric acid (2*N*, 480 mL) and extracted with ethyl acetate (4x 100 mL). The combined extracts were dried over anhydrous magnesium sulfate, filtered, and the solvent was evaporated. The residue was dried under reduced pressure, affording 65.56 g (0.25 mol, 53%) of the desired product as a yellowish liquid.

¹H-NMR (CDCl₃, 400 MHz): δ = 6.14 - 6.08 (m, 1H; =CH), 5.62 - 5.55 (m, 1H; =CH), 5.28 - 5.15 (m, 1H; CH), 4.30 - 4.08 (m, 2H; O-CH₂), 2.48 - 2.36 (m, 4H; CH₂), 2.01 - 1.90 (m, 5H; CH₂, CH₃), 1.32 - 1.25 (m, 3H; CH₃).

### Step 2: Synthesis of 1-(methacryloyloxy)propan-2-yl 5-oxo-5-thioureidopentanoate TU1

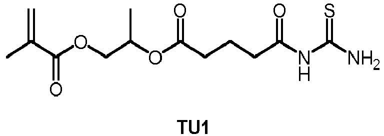

5-[(1-Methacryloyloxypropan-2-yl)oxy]-5-oxopentanoic acid (10.0 g, 38.7 mmol) was dissolved in dichloromethane (100 mL). *N*,*N*-Dimethylformamide (28 mg, 0.39 mmol) and thionyl chloride (4.61 g, 38.7 mmol) were added and the reaction mixture was stirred at ambient temperature for 22 h. Thiourea (7.37 g, 96.8 mmol) was added and the suspension was heated to reflux for 24 h. After cooling to RT, the suspension was filtered. The yellow filtrate was washed with water (2x 100 mL), saturated aqueous NaHCO₃ solution (100 mL), and brine (100 ml), dried over anhydrous sodium sulfate, filtered through a pad of silica gel, and the solvent was evaporated. The residue was dissolved in toluene (50 mL). The solution was cooled in an ice bath and stirred until a solid precipitated. *n*-Heptane (25 mL) was added and the suspension was filtered. The residue was washed with *n*-heptane/toluene 1:2 (50 mL) and dried under reduced pressure, affording 7.62 g (24.1 mmol; 62%) of the desired product as a white solid.

¹H-NMR (CDCl₃, 400 MHz): δ = 9.90 (br s, 1H; NH), 9.73 (br s, 1H; NH), 7.71 (br s, 1H; NH), 6.15 - 6.10 (m, 1H; =CH), 5.63 - 5.58 (m, 1H; =CH), 5.29 - 5.17 (m, 1H; CH), 4.32 - 4.10 (m, 2H; O-CH₂), 2.53 - 2.34 (m, 4H; CH₂), 2,05 - 1.88 (m, 5H; CH₂, CH₃), 1.29 (d, J = 6.5 Hz, 3H; CH₃).

### Example 2

### Synthesis of the acylthiourea oligomer ATUO 1

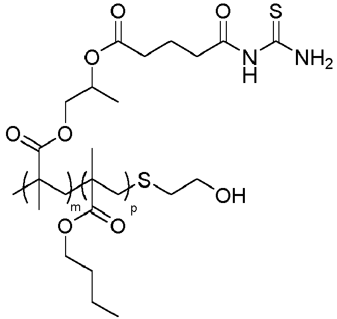

TU1 (633 mg, 2.0 mmol) and butyl methacrylate (853 mg, 6.0 mmol) were added to THF (8.6 mL) and nitrogen was bubbled through the solution for 15 min. 2-Mercaptoethanol (31.5 mg, 0.40 mmol) and AIBN (13.1 mg, 0.08 mmol) were added and the reaction mixture was heated to 70°C. The acylthiourea monomer TU1 represented 25% of the total amount of monomers. The chain transfer agent 2-mercaptoethanol was added in an amount of 5 mol-%, based on the total molar amount of monomers. After 16 h, the solvent was evaporated and the residue was dried under reduced pressure, affording 1.41 g of the desired oligomer.

Mn = 4100 g/mol (GPC); ¹H-NMR (DMSO-*d₆*, 400 MHz): δ = 11.07 (NH), 9.62 (NH), 9.36 (NH), 5.17 - 4.80 (CH- CH₃), 4.02 - 3.76 (OCH₂), 2.47 - 2.21 (CH₂), 2.13 - 0.47 (CH₂, CH₃).

### Example 3

### Synthesis of the acylthiourea oligomer ATUO 2

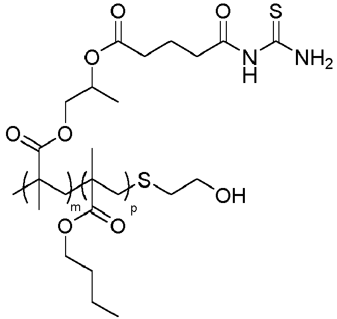

TU1 (1.27 g, 4.0 mmol) and butyl methacrylate (1.71 g, 12.0 mmol) were added to THF (14 mL) and nitrogen was bubbled through the solution for 15 min. 2-Mercaptoethanol (31.2 mg, 0.40 mmol) and AIBN (26.3 mg, 0.16 mmol) were added and the reaction mixture was heated to 70°C. The acylthiourea monomer TU1 represented 25% of the total amount of monomers. The chain transfer agent 2-mercaptoethanol was added in an amount of 2.5 mol-%, based on based on the total molar amount of monomers. After 16 h, the solvent was evaporated and the residue was dried under reduced pressure, affording 2.83 g of the desired oligomer.

Mn = 5600 g/mol (GPC); ¹H-NMR (DMSO-*d₆*, 400 MHz): δ = 11.07 (NH), 9.63 (NH), 9.36 (NH), 5.19 - 4.70 (CH- CH₃), 4.25 - 3.67 (OCH₂), 2.48 - 2.20 (CH₂), 2.12 - 0.34 (CH₂, CH₃).

### Example 4

### Synthesis of the acylthiourea oligomer ATUO 3

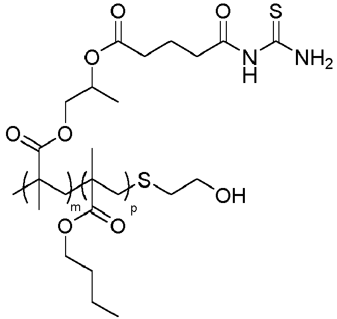

TU1 (2.53 g, 8.0 mmol) and butyl methacrylate (1.14 g, 8.0 mmol) were added to THF (16 mL) and nitrogen was bubbled through the solution for 15 min. 2-Mercaptoethanol (63.1 mg, 0.80 mmol) and AIBN (26.3 mg, 0.16 mmol) were added and the reaction mixture was heated to 70°C. The acylthiourea monomer TU1 represented 50% of the total amount of monomers. The chain transfer agent 2-mercaptoethanol was added in an amount of 5 mol-%, based on based on the total molar amount of monomers. After 16 h, the solvent was evaporated and the residue was dried under reduced pressure, affording 3.50 g of the desired oligomer.

Mn = 3800 g/mol (GPC); ¹H-NMR (DMSO-*d₆*, 400 MHz): δ = 11.06 (NH), 9.62 (NH), 9.35 (NH), 5.20 -4.80 (CH- CH₃), 4.33 - 3.66 (OCH₂), 2.68 -2.13 (CH₂), 2.11 - 0.36 (CH₂, CH₃).

### Example 5

### Synthesis of the acylthiourea oligomer ATUO 4

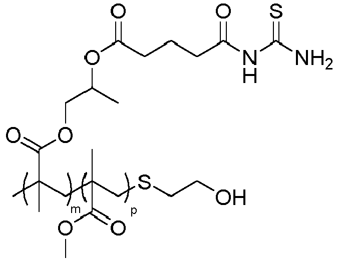

TU1 (2.53 g, 8.0 mmol) and methyl methacrylate (2.40 g, 24.0 mmol) were added to THF (28 mL) and nitrogen was bubbled through the solution for 15 min. 2-Mercaptoethanol (126 mg, 1.60 mmol) and AIBN (53 mg, 0.32 mmol) were added and the reaction mixture was heated to 70°C. The acylthiourea monomer TU1 represented 25% of the total amount of monomers. The chain transfer agent 2-mercaptoethanol was added in an amount of 5 mol-%, based on based on the total molar amount of monomers. After 16 h, the solvent was evaporated and the residue was dried under reduced pressure, affording 4.69 g of the desired oligomer.

Mn = 3200 g/mol (GPC); ¹H-NMR (DMSO-*d₆*, 400 MHz): δ = 11.08 (NH), 9.63 (NH), 9.37 (NH), 5.17 - 4.80 (CH- CH₃), 4.24 - 3.78 (OCH₂), 3.56 (OCH₃), 2.49 - 2.23 (CH₂), 2.10 - 0.55 (CH₂, CH₃).

### Example 6

### Synthesis of the acylthiourea oligomer ATUO 5

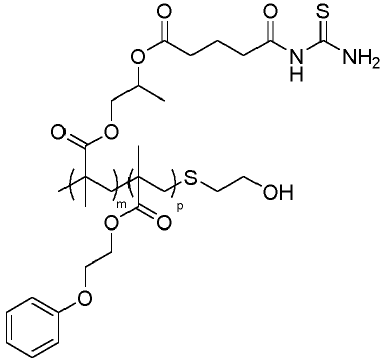

TU1 (1.27 g, 4.0 mmol) and 2-phenoxyethyl methacrylate (2.48 g, 12.0 mmol) were added to THF (21 mL) and nitrogen was bubbled through the solution for 15 min. 2-Mercaptoethanol (62.5 mg, 0.80 mmol) and AIBN (26 mg, 0.16 mmol) were added and the reaction mixture was heated to 70°C. The acylthiourea monomer TU1 represented 25% of the total amount of monomers. The chain transfer agent 2-mercaptoethanol was added in an amount of 5 mol-%, based on based on the total molar amount of monomers. After 16 h, the solvent was evaporated and the residue was dried under reduced pressure, affording 3.56 g of the desired oligomer.

Mn = 4000 g/mol (GPC); ¹H-NMR (DMSO-*d₆*, 400 MHz): δ = 11.07 (NH), 9.63 (NH), 9.37 (NH), 7.34 - 7.15 (ArH), 7.01 - 6.81 (ArH), 5.11 - 4.81 (CH- CH₃), 4.45 - 3.69 (OCH₂), 2.55 - 2.46 (CH₃), 2.44 - 2.17 (CH₂), 2.05 - 0.48 (CH₂, CH₃).

### Example 7

### Preparation of self-cure composites

The catalyst pastes C1 and C2 listed in Table 1 were prepared by homogenous mixing of the components named in the table. The pastes contained the following monomers: Bis-GMA, bis-[(2-methacryloyloxyethoxycarbonyl)amino]2,2,4(2,4,4)-trimethyl-hexane (UDMA) and 2-(2-phenylphenoxy)ethyl methacrylate (PEM). Methylhydroquinone (MEHQ) was added as a stabilizer to each paste. Cumene hydroperoxide (CHP) was used as oxidant. The barium-aluminium-borosilicate glass filler GM27884 (Schott AG, average particle size 1 µm; BET specific surface area (BET DIN ISO 9277: 2010) 3.9 m²/g; composition (% by weight): Al₂O₃: 10, B₂O₃: 10, BaO: 25 and SiO₂: 55) and a spherical SiO₂-ZrO₂ mixed oxide (Spherosil, Transparent Materials) were used as fillers.

**Table 1: Composition of catalyst pastes C1 and C2**

| **Component** | **C1 [wt%]** | **C2 [wt%]** |
|---|---|---|
| **Bis-GMA** | 11.694 | 11.394 |
| **UDMA** | 15.592 | 15.192 |
| **PEM** | 11.694 | 11.394 |
| **CHP** | 1.00 | 2.00 |
| **MEHQ** | 0.02 | 0.02 |
| **Mixed oxide Spherosil** | 10.00 | 10.00 |
| **Glass filler GM27884** | 50.00 | 50.00 |

The base pastes B1-B6 listed in Table 2 were prepared by homogeneous mixing of the components named in the table. TEMPO (CAS Registry Number 2564-83-2) was used as additional stabilizer. The barium-aluminium-borosilicate glass filler GM27884 (Schott AG, average particle size 1 µm; BET specific surface area (BET DIN ISO 9277: 2010) 3.9 m²/g; composition (% by weight ): Al₂O₃: 10, B₂O₃: 10, BaO: 25 and SiO₂: 55) and a spherical SiO₂-ZrO₂ mixed oxide (Spherosil, Transparent Materials) were used as fillers. Copper(II) acetylacetonate (Cu(acac)₂) was used as transition metal compound. For the comparative paste B6, acetylthiourea (ATU) was used as a reducing agent.

**Table 2: Composition of base pastes B1 - B6**

| **Component** | **B1 [wt%]** | **B2 [wt%]** | **B3 [wt%]** | **B4 [wt%]** | **B5 [wt%]** | **B6 [wt%]*** |
|---|---|---|---|---|---|---|
| **Bis-GMA** | 10.7892 | 11.3892 | 10.7892 | - | - | 11.811 |
| **UDMA** | 14.3856 | 15.1856 | 14.3856 | - | - | 15.748 |
| **PEM** | 10.7892 | 11.3892 | 10.7892 | - | - | 11.811 |
| **ATUO 1** | 4.00 | 2.00 | - | - | - | - |
| **ATUO 2** | - | - | 4.00 | - | - | - |
| **ATUO 3** | - | - | - | 4.00 | - | - |
| **ATUO 4** | - | - | | - | 4.00 | - |
| **ATU** | - | - | - | - | - | 0.60 |
| **Cu(acac)₂** | 0.008 | 0.008 | 0.008 | 0.008 | 0.008 | 0.002 |
| **MEHQ** | 0.02 | 0.02 | 0.02 | 0.02 | 0.02 | 0.02 |
| **TEMPO** | 0.008 | 0.008 | 0.008 | 0.008 | 0.008 | 0.008 |
| **Mixed oxide Spherosil** | 10.00 | 10.00 | 10.00 | 10.00 | 10.00 | 10.00 |
| **Glass filler GM27884** | 50.00 | 50.00 | 50.00 | 50.00 | 50.00 | 50.00 |

| | | | | | | |
|---|---|---|---|---|---|---|
| * comparative example | | | | | | |

### Example 8

### Measurement of the mechanical properties (flexural strength and modulus) of self-cure composites

The self-curing composites listed in Table 3 were prepared by mixing catalyst paste and base paste by hand in a ratio of 1/1 wt/wt. Flexural strength specimens (2 mm × 2 mm × 25 mm) were prepared using a stainless-steel mold. The mold was filled with the cement, then covered with a polyethylene film and placed in an oven at 37°C for 45 min. The specimens were removed from the mold and were stored in water at 37°C for 24 h. The measurement of the flexural strength and modulus was carried out in three-point bending tests (span: 20 mm) according to ISO4049 (Dentistry - Polymer-based filling, restorative and luting materials) with a speed of 0.8 mm min⁻¹ using a Z2.5/TS universal testing machine (Zwick, Germany).

**Table 3: Mechanical properties of self-cure composites (SCC)**

| **Self-cure composite** | **Flexural strength [MPa]** | **Flexural modulus [GPa]** |
|---|---|---|
| **SCC1 (C1 + B1)** | 85.9 ± 5.8 | 5.9 ± 0.2 |
| **SCC2 (C2 + B1)** | 93.3 ± 5.1 | 7.0 ± 0.2 |
| **SCC3 (C2 + B2)** | 107.9 ± 6.2 | 7.3 ± 0.2 |
| **SCC4 (C2 + B3)** | 98.7 ± 6.2 | 7.0 ± 0.2 |
| **SCC5 (C2 + B4)** | 90.8 ± 6.4 | 6.8 ± 0.2 |
| **SCC6 (C2 + B5)** | 100.6 ± 7.5 | 7.1 ± 0.2 |
| **SCC7 (C2 + B6*)** | 106.3 ± 7.4 | 7.0 ± 0.3 |

| | | |
|---|---|---|
| * comparative example | | |

The data in Table 3 show that the acylthiourea oligomers according to the invention resulted in composites with excellent mechanical properties. Composite SCC1 exhibited slightly lower values for flexural strength and modulus compared to the other composites, which can be attributed to the lower amount of CHP in catalyst paste C1. The other composites according to the invention exhibited similar mechanical properties as the reference material SCC7.

### Example 9

### Measurement of the working time of self-cure composites

The cements listed in Table 4 were prepared by mixing catalyst paste and base paste by hand in a ratio of 1/1 wt/wt. The working time was determined with an oscillating rheometer (Rheometer MCR 302, Anton Paar) using a plate / plate geometry, at 28.7°C. The storage modulus was recorded as a function of time (frequency = 1 Hz). A graph representing the storage modulus (logarithmic) as a function of time (non-logarithmic) was then used for the determination of the working time. The inflexion point (to be found where the slope of the function reaches its highest value) was identified. A second point on the curve was set as soon as the storage modulus started to rise (end of the stable phase). A straight line was then drawn between the two points. The point on the curve (before the inflexion point) where the tangent line is parallel to this straight line gives the working time.

**Table 4: Working times of self-cure composites (SCC)**

| **Self-cure composites** | **Working time [s]** |
|---|---|
| **SCC1 (C1 + B1)** | 269 ± 28 |
| **SCC2 (C2 + B1)** | 137 ± 2 |
| **SCC3 (C2 + B2)** | 160 ± 1 |
| **SCC4 (C2 + B3)** | 157 ± 7 |
| **SCC5 (C2 + B4)** | 198 ± 7 |
| **SCC6 (C2 + B5)** | 143 ± 7 |
| **SCC7 (C2 + B6*)** | 149 ± 6 |

| | |
|---|---|
| * comparative example | |

Table 4 shows, that the composites according to the invention had good working times. For the development of self-cure composites, working times of about 2 to 4 minutes are usually targeted. Due to its lower amount of CHP, composite SCC1 had the longest working time.

### Example 10

### Preparation of self-adhesive resin cements

The catalyst pastes C3 and C4 listed in Table 5 were prepared by homogenous mixing of the components named in the table. The pastes contained the following monomers: tetramethylxylylene diurethane-2-methylethylene glycoldi(meth)acrylate (V380), glycerol dimethacrylate (GDMA), bis-[(2-methacryloyloxyethoxycarbonyl)-amino]2,2,4(2,4,4)-trimethylhexane (UDMA) and 2-(2-phenylphenoxy)ethyl methacrylate (PEM). Methylhydroquinone (MEHQ) was added as a stabilizer. Cumene hydroperoxide (CHP) was used as oxidant. The barium-aluminium-borosilicate glass filler GM27884 (Schott AG, average particle size 1 µm; BET specific surface area (BET DIN ISO 9277: 2010) 3.9 m²/g; composition (% by weight): Al₂O₃: 10, B₂O₃: 10, BaO: 25 and SiO₂: 55) and ytterbium fluoride (200 nm, Sukgyung) were used as fillers.

**Table 5: Composition of catalyst pastes C3 and C4**

| **Component** | **C3 [wt%]** | **C4 [wt%]** |
|---|---|---|
| **V380** | 10.128 | 10.23 |
| **GDMA** | 10.128 | 10.23 |
| **PEM** | 6.752 | 6.82 |
| **UDMA** | 6.752 | 6.82 |
| **CHP** | 1.22 | 0.88 |
| **MEHQ** | 0.02 | 0.02 |
| **Ytterbium fluoride** | 15.00 | 15.00 |
| **Glass filler GM27884** | 50.00 | 50.00 |

The base pastes B7 and B8 listed in Table 6 were prepared by homogeneous mixing of the components named in the table. The base pastes contained the acidic monomer 10-methacryloyloxydecyl dihydrogen phosphate (MDP). TEMPO (CAS Registry Number 2564-83-2) was used as additional stabilizer. The barium-aluminium-borosilicate glass filler GM27884 (Schott AG, average particle size 1 µm; BET specific surface area (BET DIN ISO 9277: 2010) 3.9 m²/g; composition (% by weight): Al₂O₃: 10, B₂O₃: 10, BaO: 25 and SiO₂: 55) and ytterbium fluoride (200 nm, Sukgyung) were used as fillers. Copper(II) acetylacetonate (Cu(acac)₂) was used as transition metal compound. For the comparative paste B8, hexanoyl thiourea (HTU) was used as reducing agent.

**Table 6: Composition of base pastes B7 and B8**

| **Component** | **B7 [wt%]** | **B8* [wt%]** |
|---|---|---|
| **V380** | 9.0654 | 9.291 |
| **GDMA** | 9.0654 | 9.291 |
| **PEM** | 6.0436 | 6.194 |
| **UDMA** | 6.0436 | 6.194 |
| **MDP** | 3.00 | 3.00 |
| **ATUO 1** | 1.75 | - |
| **HTU** | - | 1.00 |
| **Cu(acac)₂** | 0.004 | 0.002 |
| **MEHQ** | 0.02 | 0.02 |
| **TEMPO** | 0.008 | 0.008 |
| **Yterbium fluoride** | 15.00 | 15.00 |
| **Glass filler GM27884** | 50.00 | 50.00 |

| | | |
|---|---|---|
| * comparative example | | |

Sulzer Mixpack two-component syringes were each filled with a catalyst paste and a base paste to produce the self-adhesive resin cements SAC1 to SAC3.

### Example 11

### Measurement of the mechanical properties (flexural strength and modulus) of self-adhesive resin cements

The self-adhesive cements listed in Table 7 were prepared by mixing catalyst paste and base paste by pressing the pastes out of the double-push syringes. The cements were filled directly into the mold for preparing the test specimens. Flexural strength and flexural modulus were determined as described in Example 8.

**Table 7: Mechanical properties of self-adhesive resin cements**

| **Self-adhesive resin cements** | **Flexural strength [MPa]** | **Flexural modulus [GPa]** |
|---|---|---|
| **SAC1 (C3 + B7)** | 125.4 ± 9.6 | 7.7 ± 0.3 |
| **SAC2 (C3 + B8)*** | 119.6 ± 7.4 | 7.0 ± 0.1 |
| **SACS (C4 + B8)*** | 112.3 ± 12.2 | 6.2 ± 0.4 |

| | | |
|---|---|---|
| * comparative example | | |

Table 7 shows that the cements had excellent mechanical properties. Cements SAC2 and SAC3 comprise an acylthiourea derivative (HTU) according to the prior art. Compared to cement SAC2, cement SAC3 had a significantly lower flexural modulus due to its lower amount of the hydroperoxide CHP.

### Example 12

### Measurement of the working time of self-adhesive resin cements

The working times of self-adhesive cements were determined as described in Example 9.

**Table 8: Working times of self-adhesive composites**

| **Self-adhesive resin cements** | **Working time [s]** |
|---|---|
| **SAC1 (C3 + B7)** | 187 ± 2 |
| **SAC2 (C3 + B8)*** | 73 ± 3 |
| **SAC3 (C3 + B8)*** | 139 ± 2 |

| | |
|---|---|
| * comparative example | |

The cement SAC1 according to the invention had a working time of 187 s. The working time of the comparative cement SAC2 was only 73 s, which is too short for many applications. Reducing the amount of hydroperoxide CHP in cement SAC3 gave an acceptable working time. However, as shown in Example 11, reducing the amount of peroxide also resulted in a significant decrease in flexural modulus. The acylthiourea oligomers according to the present invention enable the adjustment of the working time of self-adhesive cements without impairing their mechanical properties.

## Claims

1. An acylthiourea oligomer according to Formula (I) 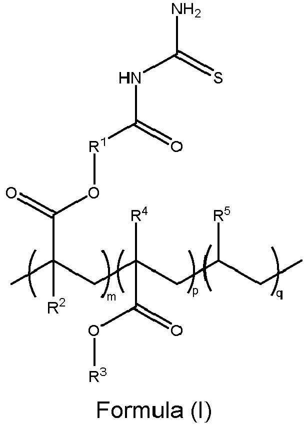 wherein:
R¹ is a branched or a linear C₁-C₁₆ alkanediyl group, preferably a C₁-C₁₂ alkanediyl group, and more preferably a C₁-C₈ alkanediyl group, that can be interrupted by one or more S atoms, O atoms, -C(O)O- groups and/or -C(O)NH- groups;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and can be interrupted by one or more S atoms, O atoms, -C(O)O- groups and/or -C(O)NH- groups;
or
a combination of the branched or the linear C₁-C₁₆ alkanediyl group and the C₄-C₁₆ cycloaliphatic group;
or
a C₇-C₂₀-alkylaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R⁸, where R⁸ is a branched or preferably linear C₁-C₂₀ alkyl group, and/or acyl groups -C(O)-R⁹, where R⁹ is a branched or preferably linear C₁-C₂₀ alkyl group;
R², R⁴ are, independently of each other, hydrogen or a methyl group;
R³ is a branched or preferably linear C₁-C₁₂ alkyl group, preferably a C₁-C₁₀ alkyl group, and more preferably a C₁-C₈ alkyl group, that can be interrupted by one or more S atoms, O atoms, -C(O)O- groups and/or -C(O)NH- groups and/or can be substituted by one or more functional groups, preferably OH, COOH and/or halogen;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, that can be interrupted by one or more S atoms, O atoms, -C(O)O- groups and/or C(O)NH- groups;
or
a C₄-C₁₀ aryl or heteroaryl group which can be unsubstituted or substituted by one or more substituents which are selected from branched or preferably linear C₁-C₂₀ alkyl groups, -O-C₁-C₁₂ alkoxy groups, ester groups -C(O)O-R¹⁰, where R¹⁰ is a branched or preferably linear C₁-C₂₀ alkyl group, a C₆-C₁₀ aryl group or a C₇-C₂₀-alkylaryl group, and/or acyl groups -C(O)-R¹¹, where R¹¹ is a branched or preferably linear C₁-C₂₀ alkyl group, a C₆-C₁₀ aryl group or a C₇-C₂₀-alkylaryl group and/or can be interrupted by one or more S atoms or O atoms;
or
a C₅-C₂₀-alkylaryl or C₅-C₂₀-alkylheteroaryl group that can be interrupted by one or more S atoms, O atoms, -C(O)O- groups and/or -C(O)NH-groups, and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R¹², where R¹² is a branched or preferably linear C₁-C₂₀ alkyl group, a C₆-C₁₀ aryl group or a C₇-C₂₀-alkylaryl group, and/or acyl groups -C(O)-R¹³, where R¹³ is a branched or preferably linear C₁-C₂₀ alkyl group, a C₆-C₁₀ aryl group or a C₇-C₂₀-alkylaryl group;
R⁵ is a phenyl or a -O(C=O)-CH₃ group;
m is an integer within a range from 1 to 100, preferably 2 to 50, most preferably 2 to 30;
p is an integer within a range from 0 to 100, preferably 2 to 50, most preferably 5 to 50;
q is an integer within a range from 0 and 100, preferably 0 to 50, most preferably 0 to 30;
m + p + q ≥ 2 and wherein
m/(m + p + q) is a value within a range from 0.1 and 1.0, preferably 0.1 to 0.5, most preferably 0.2 to 0.5.

2. The acylthiourea oligomer according to claim 1, wherein
R¹ is a branched or a linear C₁-C₁₆ alkanediyl group, preferably a C₁-C₁₂ alkanediyl group, and more preferably a C₁-C₈ alkanediyl group, that can be interrupted by one or more S atoms, O atoms, -C(O)O- groups and/or -C(O)NH- groups;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and can be interrupted by one or more S atoms, O atoms, -C(O)O- groups and/or -C(O)NH- groups;
or
a combination of branched or the linear C₁-C₁₆ alkanediyl group and the C₄-C₁₆ cycloaliphatic group;
or
a C₇-C₂₀-alkylaryl group that can be interrupted by one or more S atoms, O atoms, -C(O)O- groups and/or -C(O)NH- groups, and/or can be unsubstituted or substituted by one or more substituents selected from branched or preferably linear C₁-C₂₀ alkyl groups, branched or preferably linear O-C₁-C₁₂ alkoxy groups, ester groups -C(O)O-R⁸, where R⁸ is a branched or preferably linear C₁-C₂₀ alkyl group, and/or acyl groups -C(O)-R⁹, where R⁹ is a branched or preferably linear C₁-C₂₀ alkyl group;
R² is hydrogen atom or a methyl group;
R³ is a branched or preferably linear C₁-C₁₂ alkyl group, preferably a C₁-C₁₀ alkyl group, and more preferably a C₁-C₈ alkyl group, that can be interrupted by one or O atoms, -C(O)O- groups and/or -C(O)NH- groups and/or that can be substituted by one or more functional groups, preferably OH, COOH and/or halogen;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and can be interrupted by one or more S atoms, O atoms, -C(O)O- groups and/or -C(O)NH- groups;
or
a C₄-C₁₀ aryl or heteroaryl group which can be unsubstituted or substituted by one or more substituents selected from branched or preferably linear C₁-C₂₀ alkyl groups, -O-C₁-C₁₂ alkoxy groups, ester groups -C(O)O-R¹⁰, where R¹⁰ is either a branched or preferably linear C₁-C₂₀ alkyl group, and/or acyl groups -C(O)-R¹¹, where R¹¹ is a branched or preferably a linear C₁-C₂₀ alkyl group;
or
a C₅-C₂₀-alkylaryl or alkylheteroaryl group that can be interrupted by one or more O atoms, -C(O)O- groups and/or -C(O)NH- groups, and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R¹², where R¹² is a branched or preferably linear C₁-C₂₀ alkyl group, and/or acyl groups -C(O)-R¹³, where R¹³ is a branched or preferably linear C₁-C₂₀ alkyl group;
R⁴ is a hydrogen atom or a methyl group;
m is an integer within a range from 1 to 100, preferably 2 to 50, most preferably 2 to 30;
p is an integer within a range from 0 to 100, preferably 2 to 50, most preferably 5 and 50;
q is 0;
m + p ≥ 2 and
m/(m+p) is a value within the range from 0.1 to 1.0, preferably 0.1 to 0.5, most preferably 0.2 to 0.5.

3. The acylthiourea oligomer according to claim 2, wherein
R¹ is a branched or a linear C₁-C₁₆ alkanediyl group, preferably a C₁-C₁₂ alkanediyl group, and more preferably a C₁-C₈ alkanediyl group, that can be interrupted by one or more O atoms, -C(O)O- groups and/or -C(O)NH- groups;
R² is a hydrogen atom or a methyl group;
R³ is a branched or preferably a linear C₁-C₁₂ alkyl group, preferably a C₁-C₁₀ alkyl group, and more preferably a C₁-C₈ alkyl group, that can be interrupted by one or O atoms, -C(O)O- groups and/or -C(O)NH- groups or that can be substituted by one or more functional groups, preferably OH, COOH and/or halogen;
or
a C₄-C₁₆ cycloaliphatic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms, -C(O)O- groups and/or -C(O)NH- groups;
or
a C₅-C₂₀-alkylaryl or alkylheteroaryl group that can be interrupted by one or more O atoms, -C(O)O- groups and/or -C(O)NH- groups, and/or can be unsubstituted or substituted by one or more substituents selected from branched or preferably linear C₁-C₂₀ alkyl groups, branched or preferably linear O-C₁-C₁₂ alkoxy groups;
R⁴ is a hydrogen atom or a methyl group;
m is an integer within a range from 1 to 100, preferably 2 to 50, most preferably 2 to 30;
p is an integer within a range from 0 to 100, preferably 2 to 50, most preferably 5 to 50;
q is 0;
m + p ≥ 2 and
m/(m+p) is a value within a range from 0.1 to 1.0, preferably 0.1 to 0.5, most preferably 0.2 to 0.5.

4. A radically polymerizable composition comprising at least one acylthiourea oligomer according to any one of claims 1 to 3, at least one hydroperoxide and at least one radically polymerizable monomer.

5. The radically polymerizable composition according to claim 4, wherein the at least one hydroperoxide comprises a compound of the formula R¹⁴-(OOH)_{y}, in which R¹⁴ is an aliphatic or aromatic hydrocarbon radical and y is 1 or 2.

6. The radically polymerizable composition according to claim 5, wherein the at least one hydroperoxide is selected from t-amyl hydroperoxide, 1,1,3,3-tetramethylbutyl hydroperoxide, t-butyl hydroperoxide, t-hexyl hydroperoxide, 2,5-dimethyl-2,5-di(hydroperoxy)hexane, diisopropylbenzene monohydroperoxide, paramenthane hydroperoxide, p-isopropylcumene hydroperoxidem, cumene hydroperoxide (CHP) and a mixture thereof.

7. The radically polymerizable composition according to any one of claims 4 to 6, wherein the hydroperoxide comprises a compound according to the following Formula (IV), 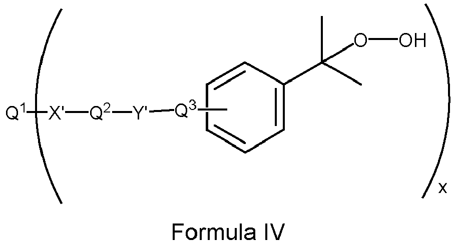 wherein the variables have the following meanings:
Q¹ a x-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR¹⁵, -Cl and -Br, wherein R¹⁵ is an aliphatic, linear or branched C₁-C₁₀ hydrocarbon radical,
X', Y' independently of each other are in each case absent, -O-, -COO-; -CONR¹⁶-, or -O-CO-NR¹⁷-, wherein R¹⁶ and R¹⁷ independently of each other represent H or a C₁-C₅ alkyl radical, preferably H, methyl and/or ethyl, particularly preferably H, and wherein X' and Y' are preferably not absent at the same time, and wherein X' and/or Y' is absent if Q² is absent,
Q² is absent, an aliphatic, linear or branched C₁-C₁₄ alkylene radical, which can be interrupted by S and/or O atoms and which can be unsubstituted or substituted by -OH, -OR¹⁸, -Cl and/or -Br, wherein R¹⁸ is an aliphatic, linear or branched C₁-C₁₀ hydrocarbon radical,
Q³ a C₁-C₃ alkylene group or is absent, preferably -CH₂- or is absent,
x 1, 2, 3 or 4, and wherein
the substitution on the aromatic compound takes place in position 2, 3 or 4, relative to the cumene hydroperoxide group.

8. The radically polymerizable composition according to any one of claims 4 to 7, wherein the at least one radically polymerizable monomer is a multifunctional (meth)acrylate, preferably a dimethacrylate or a mixture of mono- and dimethacrylates.

9. The radically polymerizable composition according to claim 8, wherein the at least one multifunctional (meth)acrylate is selected from bis-GMA (an addition product of methacrylic acid and bisphenol A diglycidyl ether), ethoxylated or propoxylated bisphenol A dimethacrylate, preferably the bisphenol A dimethacrylate with 3 ethoxy groups or 2,2-bis[4-(2-methacryloyloxypropoxy)phenyl]-propane, UDMA (an addition product of 2-hydroxyethyl methacrylate and 2,2,4-trimethylhexamethylene-1,6-diisocyanate), 2-{[(2-(methacryloyloxy)-ethoxy)carbonyl]amino} ethyl methacrylate, tetramethylxylylene diurethane ethylene glycol di(meth)acrylate, tetramethylxylylene diurethane ethylene glycol di(meth)acrylate, tetramethylxylylene diurethane-2-methylethylene glycoldi-(meth)acrylate (V380), di-, tri- or tetraethylene glycol dimethacrylate, trimethylol propanetrimethacrylate, pentaerythritol tetramethacrylate, glycerol di- and trimethacrylate, 1,4-butanediol dimethacrylate, 1,10-decanediol dimethacrylate (D3MA), bis(methacryloyloxymethyl)tricyclo-[5.2.1.02,6]decane (DCP), polyethylene glycol or polypropylene glycol dimethacrylates, polyethylene glycol 200-dimethacrylate or polyethylene glycol 400-dimethacrylate (PEG-200- or PEG-400-DMA), or 1,12-dodecanediol dimethacrylate or a mixture thereof,
and/or
wherein the at least one monofunctional (meth)acrylate is selected from benzyl and furfuryl methacrylate, 2-phenoxyethyl methacrylate, 2-(o-biphenyloxy)-ethyl methacrylate, 2-hydroxy-3-phenoxypropyl methacrylate, phenethyl methacrylate, 2-[(benzyloxycarbonyl)-amino]-ethyl methacrylate, 2-[(benzylcarbamoyl)oxy]ethyl methacrylate, 1-phenoxypropan-2-yl methacrylate, 2-(p-cumylphenoxy)ethyl methacrylate, tricyclodecane methacrylate, tricyclodecane methyl methacrylate, 2-(p-cumylphenoxy)ethyl methacrylate, cumylphenoxyethylene glycol methacrylate (CMP-1E) or a mixture thereof.

10. The radically polymerizable composition according to any one of claims 4 to 9, which additionally comprises at least one acid-group-containing radically polymerizable monomer, preferably a polymerizable carboxylic acid, phosphonic acid, a polymerizable phosphoric acid ester or an anhydride of these substances.

11. The radically polymerizable composition according to any one of claims 4 to 10, which additionally comprises a transition metal compound, preferably a compound of a transition metal which has at least two stable oxidation states, preferably a compound of copper, iron, cobalt, nickel, manganese or a mixture thereof.

12. The radically polymerizable composition according to any one of claims 4 to 11, which additionally comprises at least one organic or inorganic filler, preferably an oxide, such as SiO₂, ZrO₂ and TiO₂ or a mixed oxide of SiO₂, ZrO₂, ZnO and/or TiO₂, a nanoparticulate or microfine filler, such as pyrogenic silica or precipitated silica, glass powders, such as quartz, glass ceramic or radiopaque glass powder, preferably barium or strontium aluminium silicate glass powder, a radiopaque filler, such as ytterbium trifluoride, tantalum(V) oxide, barium sulfate, a mixed oxide of SiO₂ with ytterbium(III) oxide or tantalum(V) oxide, a ground prepolymer or a pearl polymer.

13. The radically polymerizable composition according to any one of claims 4 to 12, which comprises:
(a) 0.2 to 10.0 wt.-%, preferably 0.3 to 8.0 wt.-% and more preferably 0.5 to 5.0 wt.-% of at least one acylthiourea oligomer according to Formula (I) or preferably Formula (II),
(b) 0.1 to 5.0 wt.-%, preferably 0.1 to 4.0 wt.-% and more preferably 0.2 to 2.0 wt.-% of at least one hydroperoxide compound,
(c) 5 to 80 wt.-%, preferably 10 to 60 wt.-% and more preferably 15 to 50 wt.-% of at least one radically polymerizable monomer,
(d) 20 to 90 wt.-%, preferably 40 to 85 wt.-% and more preferably 50 to 80 wt.-% filler(s), and
(e) 0.001 to 5 wt.-%, preferably 0.01 to 3 wt.-%, additive(s),
in each case based on the total mass of the composition.

14. The radically polymerizable composition according to any one of claims 4 to 13, for therapeutic use as a dental material, as a dental cement, coating or veneering material, filling composite or luting cement.

15. Non-therapeutic use of a the radically polymerizable composition according to any one of claims 4 to 13, for the production or repair of dental restorations, such as prostheses, artificial teeth, inlays, onlays, crowns, bridges and complete dentures.
